# EUROPEAN PATENT SPECIFICATION

(11) **EP 1 711 174 B1**
(45) Date of publication and mention of the grant of the patent: **19.03.2008**
(21) Application number: 05701990.3
(22) Date of filing: 21.01.2005
(51) Int. Cl.: A61K 31/40, A61P 31/00, A61P 35/00, C07D 487/04, C07D 209/00, C07H 3/02

(54) **ADJUVANT COMPOSITIONS**
ADJUVANTE ZUSAMMENSETZUNGEN
COMPOSITIONS D'ADJUVANTS

(30) Priority: 21.01.2004 GB 0401239
(43) Date of publication of application: 18.10.2006
(73) Proprietor: Summit Corporation Plc, Abingdon Oxfordshire OX14 4RY (GB)
(72) Inventor: NASH, Robert James, Ystrad-Meurig, Ceredigion SY25 6AF (GB); WATSON, Alison Ann, Ystrad-Meurig, Ceredigion SY25 6AF (GB); EVINSON, Emma Louisa, Peterborough, Cambridgeshire PE6 7DL (GB)
(74) Representative: Hutchins, Michael Richard
(86) International application number: PCT/GB2005/000228
(87) International publication number: WO 2005/070415

(56) References cited:
- WO-A-20/04064715
- WORMALD M R ET AL: "CASUARINE-6-ALPHA-D-GLUCOSIDE FROM CASUARINA EQUISETIFOLIA AND EUGENIA JAMBOLANA" CARBOHYDRATE LETTERS, HARWOOD ACADEMIC PUBLISHERS, BASEL, CH, vol. 2, no. 3, 1996, pages 169-174, XP009028337 ISSN: 1073-5070
- NASH R J ET AL: "Casuarine: A very Highly Oxygenated Pyrrolizidine Alkaloid" TETRAHEDRON LETTERS, ELSEVIER SCIENCE PUBLISHERS, AMSTERDAM, NL, vol. 35, no. 42, 1994, pages 7849-7852, XP002274756 ISSN: 0040-4039
- WORMALD M R ET AL: "Configurational and conformational analysis of highly oxygenated pyrrolizidines: definitive identification of some naturally occurring 7a-epi-alexines" TETRAHEDRON: ASYMMETRY, ELSEVIER SCIENCE PUBLISHERS, AMSTERDAM, NL, vol. 9, no. 14, 17 July 1998 (1998-07-17), pages 2549-2558, XP004131419 ISSN: 0957-4166
- BELL A A ET AL: "Synthesis of Casuarines [Pentahydroxylated Pyrrolizidines] by Sodium Hydrogen Telluride-Induced Cyclisations of Azidodimesylates" TETRAHEDRON LETTERS, ELSEVIER SCIENCE PUBLISHERS, AMSTERDAM, NL, vol. 38, no. 33, 18 August 1997 (1997-08-18), pages 5869-5872, XP004085896 ISSN: 0040-4039
- KATO A ET AL: "Australine and related alkaloids: easy structural confirmation by <13>C NMR spectral data and biological activities" TETRAHEDRON: ASYMMETRY, ELSEVIER SCIENCE PUBLISHERS, AMSTERDAM, NL, vol. 14, no. 3, 7 February 2003 (2003-02-07), pages 325-331, XP004405423 ISSN: 0957-4166

## Description

### Field of the Invention

The present invention relates to adjuvants, and in particular to alum-based adjuvants and vaccines based thereon.

### Background to the Invention

### Immunity

When the Immune system is challenged by a foreign antigen it responds by launching a protective response. This response is characterized by the coordinated interaction of both the innate and acquired immune systems. These systems, once thought to be separate and independent, are now recognized as two interdependent parts that when integrated fulfil two mutually exclusive requirements: speed (contributed by the innate system) and specificity (contributed by the adaptive system).

The innate immune system serves as the first line of defence against invading pathogens, holding the pathogen in check while the adaptive responses are matured. It is triggered within minutes of infection in an antigen-independent fashion, responding to broadly conserved patterns in the pathogens (though it is not non-specific, and can distinguish between self and pathogens). Crucially, it also generates the inflammatory and co-stimulatory milieu (sometimes referred to as the *danger signal*) that potentiates the adaptive immune system and steers (or polarizes it) towards the cellular or humoral responses most appropriate for combating the infectious agent (discussed in more detail below).

The adaptive response becomes effective over days or weeks, but ultimately provides the fine antigenic specificity required for complete elimination of the pathogen and the generation of immunologic memory. It is mediated principally by T and B cells that have undergone germline gene rearrangement and are characterized by an exquisite specificity and long-lasting memory. However, it also involves the recruitment of elements of the innate immune system, including professional phagocytes (macrophages, neutrophils etc.) and granulocytes (basophils, eosinophils etc.) that engulf bacteria and even relatively large protozoal parasites. Once an adaptive immune response has matured, subsequent exposure to the pathogen results in its rapid elimination (usually before symptoms of infection become manifest) because highly specific memory cells have been generated that are rapidly activated upon subsequent exposure to their cognate antigen.

### Interdependence of innate and adaptive responses

It is now thought that the earliest events following pathogen invasion are effected by cellular components of the innate immune system. The response is initiated when resident tissue macrophages and dendritic cells (DCs) encounter pathogen and become activated by signals generated by interaction between pattern-recognition receptors (PRRs) and the pathogen-associated molecular patterns (PAMPs) shared by large groups of microorganisms. The activated macrophages and DCs are stimulated to release various cytokines (including the chemokines IL-8, MIP-1α and MIP-1β), which constitute the "danger signal" and triggers an influx of NK cells, macrophages, immature dendritic cells into the tissues.

Loaded with antigen, the activated DCs then migrate to lymph nodes. Once there, they activate immune cells of the adaptive response (principally naïve B- and T-cells) by acting as antigen-presenting cells (APCs). The activated cells then migrate to the sites of Infection (guided by the "danger signal") and once there further amplify the response by recruiting cells of the innate immune system (including eosinophils, basophils, monocytes, NK cells and granulocytes). This cellular trafficking is orchestrated by a large array of cytokines (particularly those of the chemokine subgroup) and involves immune cells of many different types and tissue sources (for a review, see Luster (2002), Current Opinion in Immunology 14: 129-135).

### Polarization of the adaptive immune response

The adaptive immune response is principally effected *via* two independent limbs: cell-mediated (type 1) immunity and antibody-mediated or humoral (type 2) immunity.

Type 1 immunity involves the activation of T-lymphocytes that either act upon infected cells bearing foreign antigens or stimulate other cells to act upon infected cells. This branch of the immune system therefore effectively contains and kills cells that are cancerous or infected with pathogens (particularly viruses). Type 2 immunity involves the generation of antibodies to foreign antigens by B-lymphocytes. This antibody-mediated branch of the immune system attacks and effectively neutralizes extracellular foreign antigens.

Both limbs of the immune system are important in fighting disease and there is an increasing realization that the type of immune response is just as important as its intensity or its duration. Moreover, since the type 1 and type 2 responses are not necessarily mutually exclusive (in many circumstances an effective immune response requires that both occur in parallel), the *balance* of the type1/type 2 response (also referred to as the *Th1:Th2 response ratio*/*balance* by reference to the distinct cytokine and effector cell subsets involved in the regulation of each response - see below) may also play a role in determining the effectiveness (and repercussions) of the immune defence.

In many circumstances the immune response is skewed heavily towards a type 1 or type 2 response soon after exposure to antigen. The mechanism of this type1/type 2 skewing or polarization is not yet fully understood, but is known to involve a complex system of cell-mediated chemical messengers (cytokines, and particularly chemokines) in which the type1/type 2 polarization (or balance) is determined, at least in part, by the nature of the initial PRR-PAMP interaction when the DCs and macrophages of the innate immune system are first stimulated and subsequently by the cytokine milieu in which antigen priming of naïve helper T cells occurs.

Two cytokines in particular appear to have early roles in determining the path of the immune response. Interleukin-12 (IL-12), secreted by macrophages, drives the type 1 response by stimulating the differentiation of Th1 cells, the helper cells that oversee the type 1 response. Another macrophage cytokine, interleukin-10 (IL-10) inhibits this response, instead driving a type 2 response.

The type 1 and type 2 responses can be distinguished inter alia on the basis of certain phenotypic changes attendant on priming and subsequent polarization of naïve helper T cells. These phenotypic changes are characterized, at least in part, by the nature of the cytokines secreted by the polarized helper T cells.

Th1 cells produce so-called *Th1 cytokines,* which include one or more of TNF, IL-1, IL-2, IFN-gamma, IL-12 and/or IL-18. The Th1 cytokines are involved in macrophage activation and Th1 cells orchestrate Type 1 responses. In contrast, Th2 cells produce so-called *Th2 cytokines*, which include one or more of IL-4, IL-5, IL-10 and IL-13. The Th2 cytokines promote the production of various antibodies and can suppress the type 1 response.

The involvement of Th1 and Th2 cells and cytokines in type 1:type 2 immune response polarization has given rise to the terms *Th1 response* and *Th2 response* being used to define the type 1 and type 2 immune responses, respectively. Thus, these terms are used interchangeably herein.

### Vaccines

Vaccines present antigens (typically foreign antigens or neoantigens) from disease causing agents to the immune system of a host to prompt that host to mount the therapeutic and/or prophylactic immune responses described above.

Most vaccines in use today provide both the antigenic components of a pathogen (required for specificity) and the PAMPs capable of activating the PRR of innate immune cells (required for potentiating a response) respectively. Such vaccines are based on killed or attenuated forms of the microbes (typically bacterial cells, viral particles or parts thereof) that cause the disease (or in the case of cancer vaccines, from the malignant cells of the tumour) and can be extremely potent therapeutic and prophylactic agents.

However, the presence of non-essential (and sometimes dangerous or undesirable) components in these poorly characterized killed or attenuated vaccines can lead to poor side-effect profiles and has produced a negative regulatory climate that limits the development and introduction of new whole cell vaccines. This has encouraged considerable efforts to refine vaccine components and has fostered the development of *subunit vaccines, conjugate vaccines* and *DNA vaccines.*

Subunit vaccines are based on synthetic or isolated antigens created using (bio)chemical and/or recombinant techniques (e.g. recombinant peptides, protein and/or carbohydrate synthesis or purification). Conjugate vaccines involve the linkage (usually by chemical crosslinking) of relatively non-immunogenic (usually carbohydrate-based) antigens to more strongly immunogenic carrier proteins. DNA vaccines deliver the antigen(s) in the form of encoding nucleic acid and so rely on endogenous expression of the coding DNA after administration. Such vaccines normally require an appropriate vector (e.g. plasmid, viral or lipid vesicle) to deliver the coding nucleic acid to the appropriate compartment of the host.

Although subunit, conjugate and DNA vaccines are generally well tolerated with good side effect profiles, they generally lack the PAMPs that are required for innate immune activation. Thus, they typically exhibit far less potency when compared to the complex antigen mixtures present in many of the established attenuated/killed vaccines: the relatively simple nature of the low-molecular weight synthetic antigens, though devoid of potentially harmful contaminants, are generally themselves not very immunogenic.

Vaccine development is also driven by other considerations. For example, the efficacy of any given vaccines may also be compromised by the status of the host. Old or immunocompromised hosts are poor responders to most vaccines: in certain immunocompromised individuals (e.g. kidney dialysis patients and alcoholics) the rate of non-response can approach 50%. Non-response may also arise from MHC-restriction: between 5 and 10% of individual are hon-respbnders or hypo-responders to the subunit HBsAg vaccine and this appears to reflect a failure in this host population to recognize T-helper epitopes.

Recent world events have demonstrated that bioterrorism is a very real threat. From a medical perspective, novel and broadly effective defensive strategies will be needed to prevent or treat a potential man made epidemic. The threat of intentional use of biological weapons provides the impetus to improve existing biodefence vaccines (e.g. anthrax, smallpox) and to create new ones (e.g. plague, tularemia).

Therefore, a challenge of modern vaccine design is to develop strategies for efficient targeting of both the innate and adaptive immune response to mimic more closely a natural infection and achieve potent responses to defined antigens, while limiting toxicity. A great deal of the effort in this sphere is currently centred on *adjuvants -* immune response enhancers which are now seen to be essential for making new vaccine types viable, making existing vaccines more efficacious and making those that are expensive to manufacture or in scarce supply go further.

### Adjuvants

An adjuvant is any compound or composition that increases the strength and/or duration of an immune response to a foreign antigen relative to that elicited by the antigen alone. Key functional characteristics of an adjuvant therefore include its ability to enhance an appropriate immune response to the target antigen, long-term safety in widespread application, and flexibility in use with different antigen/disease applications.

The history of adjuvant discovery can be characterized as a mix of alchemy and luck, since the mechanisms of action of most adjuvants remain only partially understood. Although many different adjuvants are known, the only adjuvant presently licensed for human use with any antigen is *alum* (a term defining a group of aluminum salts which includes aluminum hydroxide and aluminum phosphate).

Although the mechanism of action of alum is not fully understood, it appears that part of its activity arises from the so-called *depot effect*. Small antigens, injected into the bloodstream, can quickly become degraded in the liver or filtered by the kidneys. Alum, and other adjuvants exhibiting a depot affect (see below), can precipitate soluble antigens (or otherwise increase their residence time) in the area near the injection site, where macrophages and dendritic cells can process them. In this way they act as antigen delivery/targeting vehicles (and not necessarily directly as immune stimulants per se), mediating a more effective presentation of antigen to the innate immune system.

However, recent work has shown that alum also has a direct immune potentiating effect *via* cytokine-mediated control of Th1/Th2 responses (see e.g. Brewer et al. (1999) The Journal of Immunology, 163: 6448-6454).

Whatever its biological basis, the immune stimulation exhibited by alum is strongly polarized towards a type 2 response (i.e. alum effectively has only type 2 adjuvant properties). This is a major limitation, since it is now recognized that type 1 immune responses (particularly the induction of T-helper type 1 (Th-1) cells and cytotoxic T-lymphocytes) are essential for generating protective immunity against many infectious agents. For example, many viral, bacterial, protozoal and intracellular infections (including diseases mediated by intracellular parasites and pathogens, such as leishmania and malaria) require a strong cell-mediated immune response for adequate protection.

Thus, alum is unsuitable for use with many modern vaccines designed to stimulate a type 1 response. For example, it has been reported that alum does not improve the effectiveness of whooping cough and typhoid vaccines and provides only a slight adjuvant effect with adenovirus vaccines. Thus the application of alum in clinical vaccines has largely been limited to situations where protection is afforded by type 2 responses, in particular the production of neutralizing antibodies.

These limitations of alum, together with the more general needs driving the development of vaccine performance discussed above, have prompted efforts to discover new vaccine adjuvants with greater potency and/or the ability to facilitate a predetermined balance of type 1 (cellular) and type 2 (antibody) responses. However, although several candidates are in various stages of development, these candidates suffer from problems associated with side effects and an undesirable sensitivity to small changes in structure (which can occur during formulation, on storage or *in vivo* after administration and which can dramatically increase toxicity and/or decrease efficacy). Some of these adjuvants are described In more detail below.

Pollock et al. (2003) Immunology 108: 137-143 describe adjuvant systems comprising alum and certain cytokines. Specifically, they show that the adsorption of IL-12 (with or without IL-18) to alum gels can influence the Th1/Th2 profile of the induced response. In particular, alum gel with adsorbed IL-12 exhibits a more balanced Th1:Th2 profile, the IL-12 acting to polarize the alum-induced Th2 response towards a Th1 response. Although not active in the absence of IL-12, the use of IL-18 as a second auxiliary adjuvant produced a synergistic effect, allowing the IL-12 to further augment the type 1 response and promote an even more pronounced Th1 polarization. The authors note that the more balanced profile of the alum/cytokine adjuvant systems (resulting from the cytokine-induced Th1 polarization/augmentation of the alum-induced Th2 response) may have applications in improving the performance of alum-based adjuvants, but note that the reactogenicity of IL-12 is likely to be an obstacle to the application of this finding in human vaccines.

US5976539 describes the potential use of interleukin-12 (IL-12) as an adjuvant and highlights its potential as an activator of type 1 immunity. Its potential in vaccines against infections requiring enhanced cell mediated immune responses for effective protection against infection by a pathogen is emphasised. However, IL-12 is expensive to produce and formulate, and its use in humans is fraught with difficulties arising from its reactogenicity and pleiotropic effects *in vivo* (which can give rise to dosing problems and unexpected side-effects).

WO8809336 describes various immunologically active saponin fractions having adjuvant activity (known generically in the art as *saponin adjuvants*). The fractions have been used successfully in veterinary practice and are derived from the bark of the South American tree *Quillaja saponaria*. Substantially pure saponins are shown to have adjuvant activity at a much lower concentration than heterogeneous saponin preparations, and do not exhibit their toxic effects. For example QS-21, also known as QA21, is an HPLC purified fraction disclosed (as QA21) in US5057540. QS-21 saponin adjuvants stimulate both type 1 and type 2 immune responses and have been used in recent large-scale clinical trials of both prophylactic and therapeutic vaccines with great success.

WO9014837 describes various submicron oil-in-water emulsions and their use as adjuvants. In particular, this documents describes adjuvant compositions comprising a metabolizable oil and an emulsifying agent, wherein the oil and the emulsifying agent are present in the form of an oil-in-water emulsion having oil droplets substantially all of which are less than 1 micron in diameter. One of the emulsions described, a microfluidized emulsion system consisting of polysorbate 80 and sorbitan trioleate dubbed MF59, has recently been approved for use in an influenza vaccine in Italy. However, MF59, like alum, stimulates a strongly polarized type 2 response.

Bacterial lipopolysaccharide endotoxin (LPS), and specifically its active moiety, lipid A, has been shown to be a potent Immunologic adjuvant. Native dephosphorylated lipid A (DPL) is reactogenic and pyrogenic; however, simple chemical modifications to DPL result in nontoxic preparations which retain the adjuvant activity of DPL. Clinical trials have been conducted with lipid A purified from *Salmonella minnesota* R595 and detoxified by removal of the phosphate group from the reducing end of the disaccharide back-bone of DPL by acid hydrolysis, to produce monophosphoryl lipid A (MPL). Mono-phosphoryl lipid A may be further detoxified by 3-O-deacylation with mild alkaline hydrolysis and may be encapsulated in liposomes composed of phospholipids and cholesterol.

Bacterial DNA, but not vertebrate DNA, has direct immunostimulatory effects on peripheral blood mononuclear cells due to the presence of unmethylated CpG dinucleotides. These are present at relatively high frequency in bacterial DNA but under-represented in vertebrate DNA. US2003091599 describes oligonucleotides adjuvants having at least one unmethylated CpG dinucleotide and shows that CpG DNA induces a Th-1 pattern of cytokine production dominated by IL-12 and IFN-gamma, with little secretion of Th2 cytokines. Thus, CpG directly activates monocytes, macrophages, and dendritic cells to secrete a variety of cytokines, including high levels of lL-12. These cytokines stimulate natural killer (NK) cells to secrete gamma-interferon and also increase their lytic activity.

Studies have also been carried out with an emulsion consisting of squalene, Pluronic L121 block polymer and Tween 80 in phosphate-buffered saline (*SAF-m adjuvant*), both alone and in combination with threonyl muramyl dipeptide (Termutide^{™}; N-acetylmuramyl-L-threonyl-D-Isoglutamine; MDP). The studies showed that the SAF-m adjuvant, in combination with influenza, HSV gD2, and HIV-1 immunogens, exhibited greater adjuvant potency than alum and (in some cases) MF59.

Muramyl dipeptide (MDP) represents the minimal unit of the mycobacterial cell wall complex that generates the adjuvant activity observed with complete freund's adjuvant (Ellouz et al. (1974) Biochem. Biophys. Res. Comm., 59:1317). Many synthetic analogues of MDP have been generated that exhibit a wide range of adjuvant potency and side effects (reviewed in Chedid et al. (1978) Prog. Allergy, 25:63). Three analogues that may be especially useful as vaccine adjuvants are threonyl derivatives of MDP, n-butyl derivatives and lipophilic derivative of muramyl tripeptide. These compounds are reported to effectively stimulate both type 1 and type 2 responses with low levels of toxicity.

One promising lipophilic derivative of MDP is N-acetylmuramyl-L-alanyl-D-isoglutaminyl-L-alanine-2-[1,2-dipalmitoyl-sn-glycero-3-3(hydroxyphosphoryloxy)]ethylamide (MTP-PE). This muramyl tripeptide has phospholipid tails that allow association of the hydrophobic portion of the molecule with a lipid environment while the muramyl peptide portion associates with the aqueous environment. Thus the MTP-PE itself can act as an emulsifying agent to generate stable oil in water emulsions.

Thus, there is a need for further potent, low-toxicity adjuvants and adjuvant systems. There is also a need for further balanced adjuvants and adjuvant systems which stimulate both limbs of the immune system and which therefore improve the safety and efficacy of existing vaccines and are suitable for use with the weakly immunogenic synthetic and cell-based vaccines now coming into widespread use. There is also a need for improved adjuvants and adjuvant systems that improve the performance of vaccines in old or immunocompromised patient populations.

An adjuvant or adjuvant system that rendered lower doses more effective, moreover, would allow for cheaper vaccines in cases in which the antigen is expensive to produce (as is often the case with recombinant, DNA or subunit vaccines). It could also stretch supplies in emergencies (as might arise, for example, during an epidemic). Such dose *sparing* properties are of particular importance in the case of conjugate vaccines, where difficult chemistry often limits antigen dose size. Dose sparing is also important in circumstances where several different antigens are co-administered in a single shot.

### Alkaloids

The term *alkaloid* is used herein *sensu stricto* to define any basic, organic, nitrogenous compound which occurs naturally in an organism. The term *alkaloid* is also used herein *sensu lato* to define a broader grouping of compounds which include not only the naturally occurring alkaloids, but also their synthetic and semi-synthetic analogues and derivatives.

Most known alkaloids are phytochemicals, present as secondary metabolites in plant tissues (where they may play a role in defence), but some occur as secondary metabolites in the tissues of animals, microorganisms and fungi. There is growing evidence that the standard techniques for screening microbial cultures are inappropriate for detecting many classes of alkaloids (particularly highly polar alkaloids, see below) and that microbes (including bacteria and fungi, particularly the filamentous representatives) will prove to be an important source of alkaloids as screening techniques become more sophisticated.

Structurally, alkaloids exhibit great diversity. Many alkaloids are small molecules, with molecular weights below 250 Daltons. The skeletons may be derived from amino acids, though some are derived from other groups (such as steroids). Others can be considered as sugar analogues. It is becoming apparent (see Watson et al. (2001) Phytochemistry 56: 265-295) that the water soluble fractions of medicinal plants and microbial cultures contain many interesting novel polar alkaloids, including many carbohydrate analogues. Such analogues include a rapidly growing number of polyhydroxylated alkaloids.

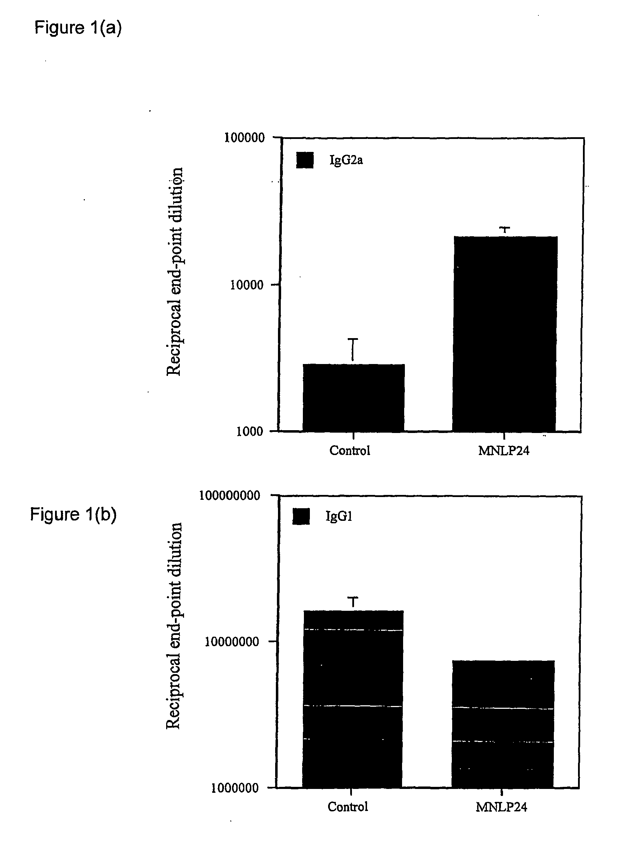

Most alkaloids are classified structurally on the basis of the configuration of the N-heterocycle. Examples of some important alkaloids and their structures are set out in Kutchan (1995) The Plant Cell 7:1059-1070. Watson et al. (2001) Phytochemistry 56: 265-295 have classified a comprehensive range of polyhydroxylated alkaloids *inter alia* as piperidine, pyrroline, pyrrolidine, pyrrolizidine, indolizidine and nortropanes alkaloids (see Figs. 1-7 of Watson *et al*. (2001), the disclosure of which is incorporated herein by reference).

Watson *et al*. (2001), *ibidem* also show that a functional classification of at least some alkaloids is possible on the basis of their glycosidase inhibitory profile: many polyhydroxylated alkaloids are potent and highly selective glycosidase inhibitors. These alkaloids can mimic the number, position and configuration of hydroxyl groups present in pyranosyl or furanosyl moieties and so bind to the active site of a cognate glycosidase, thereby inhibiting it. This area is reviewed in Legler (1990) Adv. Carbohydr. Chem. Biochem. 48: 319-384 and in Asano et al. (1995) J. Med. Chem. 38: 2349-2356.

It has long been recognized that many alkaloids are pharmacologically active, and humans have been using alkaloids (typically in the form of plant extracts) as poisons, narcotics, stimulants and medicines for thousands of years. The therapeutic applications of polyhydroxylated alkaloids have been comprehensively reviewed in Watson *et al.* (2001), *ibidem*: applications include cancer therapy, immune stimulation, the treatment of diabetes, the treatment of infections (especially viral infections), therapy of glycosphingolipid lysosomal storage diseases and the treatment of autoimmune disorders (such as arthritis and sclerosis).

Both natural and synthetic mono- and bi-cyclic nitrogen analogues of carbohydrates are known to have potential as chemotherapeutic agents. Alexine (1) and australine (2) were the first alkaloids to be isolated with a carbon substituent at C-3, rather than the more common C-1 substituents characteristic of the necine family of pyrrolizidines.

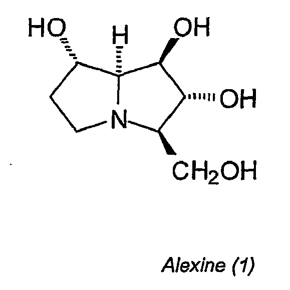

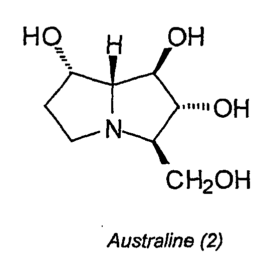

The alexines occur in all species of the genus Alexa and also in the related species *Castanospermum australe.* Stereoisomers of alexine, including 1,7a-diepialexine (3), have also been isolated (Nash et al. (1990) Phytochemistry (29) 111) and synthesised (Choi et al. (1991) Tetrahedron Letters (32) 5517 and Denmark and Cottell (2001) J. Org. Chem. (66) 4276-4284).

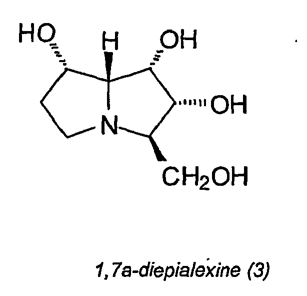

Because of the reported weak *in vitro* antiviral properties of one 7,7a-diepialexine (subsequently defined as 1,7a-diepialexine), there has been some interest in the isolation of the natural products and the synthesis of analogues.

As an indolizidine alkaloid (and so structurally distinct from the alexines), swainsonine (4) is a potent and specific inhibitor of α-mannosidase and is reported to have potential as an antimetastic, tumour anti-proliferative and immunoregulatory agent (see e.g. US5650413, WO00/37465, WO93/09117).

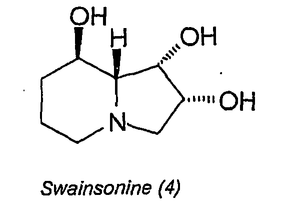

Another indolizidine alkaloid, castanospermine (5), is a potent α-glucosidase inhibitor. This compound, along with certain 6-O-acyl derivatives (such as that known as *Bucast* (6)), has been reported to exhibit anti-viral and antimetastic activities.

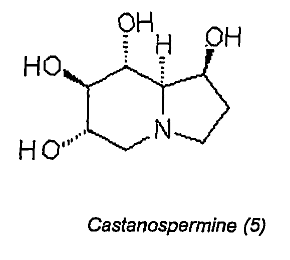

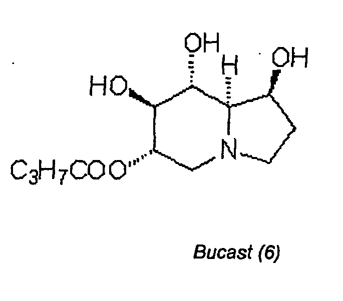

The effect of variation in the size of the six-membered ring of swainsonine on its glycosidase inhibitory activity has been studied: derivatives (so-called "ring contracted swainsonines") have been synthesised. However, these synthetic derivatives (1S, 2R, 7R, 7aR)-1,2,7-trihydroxy(7) and the 7S-epimer (8)) were shown to have much weaker inhibitory activity relative to swainsonine itself (see US5075457).

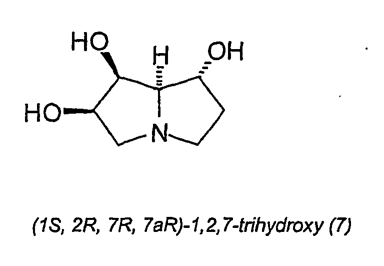

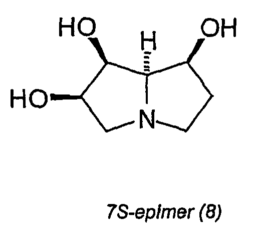

Another compound, 1α,2α,6α,7α,7αβ-1,2,6,7-tetrahydroxy(9) is an analogue of 1,8-diepiswainsonine and described as a "useful" inhibitor of glycosidase enzymes in EP0417059.

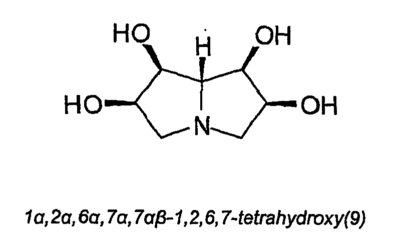

Casuarine, (1R,2R,3R,6S,7S,7aR)-3-(hydroxymethyl)-1,2,6,7-tetrahydroxy(10) is a highly oxygenated bicyclic alkaloid that can be regarded as a more highly oxygenated analogue of the 1,7a-diepialexine (shown in 3) or as a C(3) hydroxymethyl-substituted analogue of the 1α,2α,6α,7α,7αβ-1,2,6,7-tetrahydroxy(shown in 9).

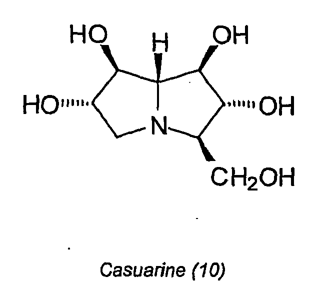

Casuarine can be isolated from several botanical sources, including the bark of *Casuarina eguisetifolia* (Casuarinaceae), the leaves and bark of *Eugenia jambolana* (Myrtaceae) and *Syzygium guineense* (Myrtaceae) (see e.g. Nash et al. (1994) Tetrahedron Letters (35) 7849-7852). Epimers of casuarine, and probably casuarine itself, can be synthesised by sodium hydrogen telluride-induced cyclisation of azidodimesylates (Bell et al. (1997) Tetrahedron Letters (38) 5869-5872).

*Casuarina equisetifolia* wood, bark and leaves have been claimed to be useful against diarrhoea, dysentery and colic (Chopra et al. (1956) Glossary of Indian Medicinal Plants, Council of Scientific and Industrial Research (India), New Delhi, p. 55) and a sample of bark has recently been prescribed in Western Samoa for the treatment of breast cancer. An African plant containing casuarine (identified as *Syzygium* guineense) has been reported to be beneficial in the treatment of AIDS patients (see Wormald et al. (1996) Carbohydrate Letters (2) 169-174).

The casuarine-6-α-glucoside (casuarine-6-α-D-glucopyranose, 11) has also been isolated from the bark and leaves of *Eugenia jambolana* (Wormald et al. (1996) Carbohydrate Letters (2) 169-174).

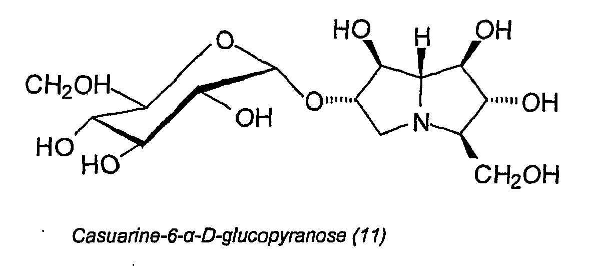

*Eugenia jambolana* is a well-known tree in India for the therapeutic value of its seeds, leaves and fruit against diabetes and bacterial infections. Its fruit have been shown to reduce blood sugar levels in humans and aqueous extracts of the bark are claimed to affect glycogenolysis and glycogen storage in animals (Wormald et al. (1996) Carbohydrate Letters (2) 169-174).

Some pyrrolidine alkaloids appear to be fairly widespread secondary metabolites: for example, 2R,5R-dihydroxymethyl-3R,4R-dihydroxypyrrolidine (DMDP) (12) and 1,4-dideoxy-1,4-imino-D-arabinitol (D-AB1) (13) have been isolated from species of both temperate and tropical plants from quite unrelated families, and DMDP is also produced by a species of the filamentous bacterium *Streptomyces.*

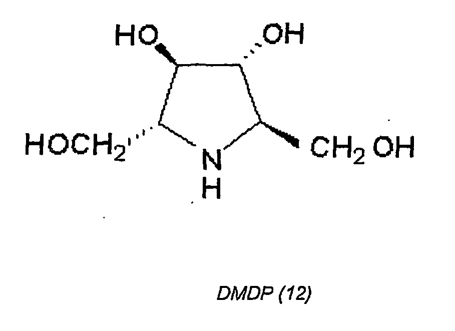

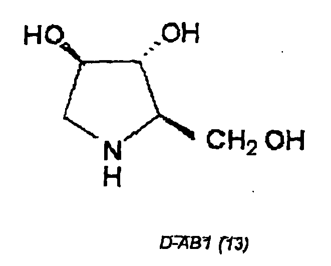

DMDP has been shown to have nematocidal activity: WO 92/09202 describes the use of the compound in controlling diseases caused by parasitic nematodes in both plants and mammals.

### Summary of the Invention

The present invention is based, at least in part, on the surprising discovery that certain alkaloids can stimulate a Th1 (type 1) immune response when co-administered with an antigen. When co-administered with a type 2 (Th2) adjuvant, such as alum or MF59, the alkaloids of the invention can polarize, skew or shift the type 2 Immune response from Th2 towards Th1, so effectively augmenting the alum/MF59-Induced Th2 response with a Th1 response to produce a balanced adjuvant activity profile.

Thus, according to the present invention there is provided the use as defined in the claims.

Preferably, the adjuvant composition further comprises one or more auxiliary edjuvant(s). In such embodiments, the composition of the invention constitutes an *adjuvant system*, as herein defined.

The auxiliary adjuvant in the adjuvant systems of the Invention may comprise a Th1, T2 or balanced adjuvant, or where two or more auxiliary adjuvants are employed, mixtures of Th1, Th2 or balanced adjuvants.

In particularly preferred embodiments, the auxiliary adjuvant is a type 2 adjuvant, for example alum or MF59. By varying the relative quantities of alkaloid and type 2 adjuvants, balanced adjuvants with a fixed Th1:Th2 ratio preselected from a wide spectrum may be produced.

Most preferred are adjuvant systems comprising the alkaloid of the invention and alum as auxiliary adjuvant. In such embodiments, the alum may be present as the sole auxiliary adjuvant, so that the adjuvant system consists (or consists essentially) of the Th1-activating alkaloid and alum. Such adjuvants may function as balanced adjuvants, since the alum-induced type 2-immune response may be augmented by a type 1 response contributed by the alkaloid of the invention. Moreover, the Th1:Th2 balance produced by such compositions may be predetermined (and indeed rationally preselected according to the nature of the ultimate vaccine target) by varying the relative amounts of alkaloid and alum. In this way, balanced adjuvants with a preselected Th1:Th2 ratio anywhere in the {type 1 ↔ type 2} continuum may be produced.

The alkaloid and auxiliary adjuvant (e.g. alum) may be physically associated: for example, the alkaloid may be physically adsorbed onto alum gel. Alternatively, the auxiliary adjuvant and alkaloid may be physically dissociated and merely co-administered (as herein defined).

Any suitable form of alum may be used according to the invention. Suitable forms include aluminium salts, for example aluminium hydroxide, aluminium phosphate or mixtures of aluminium hydroxide and aluminium phosphate. Particularly preferred is the aluminum hydroxide commercially available as Alhydrogel^{™}.

The composition of the invention may further comprise a pharmaceutically acceptable excipient. Suitable excipients are described in more detail below (in the section entitled "Formulation").

Any suitable auxiliary adjuvant or combination thereof may be used. Particularly preferred are synergistic combinations of one or more auxiliary adjuvants in which the Th1/Th2 stimulation and/or polarization is greater than the sum of that produced by the alkaloid and individual adjuvant(s) alone. Also preferred are auxiliary adjuvant(s) which, when present with the alkaloid in an adjuvant system, functionally complement the Th1 activity of the alkaloid to augment the alkaloid-induced type 1 response with a counter-balancing type 2 response. However, for some applications it is desirable to skew or polarize the adjuvant immune response as far as possible in the Th1 direction, and the selection of type 1 auxiliary adjuvant(s) may be indicated in such cases. Such strongly polarized type 1 adjuvant systems may find particular application in therapeutic vaccines, and especially in applications where a strong CTL response is required (as is the case, for example, in the treatment of cancers and diseases caused by certain intracellular pathogens).

Suitable for use as auxiliary adjuvants according to the invention are cytokines (for example, Th1 cytokines, e.g. IL-12, IL-18 and/or IFN-gamma). Other suitable auxiliary adjuvants are those which function as carriers and/or depot-forming agents (such as lipid vesicles or liposomes, various gels or emulsions, for example the Seppic ISA series of Montanide adjuvants and Provax^{™}, the latter being an oil-in-water emulsion containing a stabilizing detergent and a micelle-forming agent). Yet others include virus-like particles, virosomes and ISCOM® matrices and particles. ISCOM® particles provide both adjuvant and antigen delivery properties. The particles comprise saponins, cholesterol and phospholipids. Particles without an incorporated or associated antigen are called ISCOMATRIX®. When antigens are incorporated into the particle or associated with preformed ISCOMATRIX® it is called an ISCOM®.

Other suitable auxiliary adjuvants for use according to the invention include saponins (particularly QS-21), submicron oil-in-water emulsions (particularly MF59) and CpGs.

Yet other suitable auxiliary adjuvants for use according to the invention include lipid A derivatives (for example poly[di(carboxylatophenoxy)phosphazene (PCPP) derivatives of lipopolysaccharides, such as monophosphorlyl lipid (MPL)). SB-AS2 (SmithKline Beecham adjuvant system #2, an oil-in-water emulsion containing MPL and QS21) may also be used, as may SB-AS4 (SmithKline Beecham adjuvant system #4, which contains alum and MPL).

Other suitable auxiliary adjuvants for use according to the invention include MDPs (for example any of the many synthetic analogues reviewed in Chedid et al. (1978) Prog. Allergy, 25:63, including threonyl derivatives of MDP, n-butyl derivatives and lipophilic derivative of muramyl tripeptide (for example, N-acetylmuramyl-L-alanyl-D-isoglutaminyl-L-alanine-2-[1,2-dipalmitoyl-sn-glycero-3-3(hydroxyphosphoryloxy)]ethylamide (MTP-PE). An adjuvant system comprising a combination of an emulsion and an MDP may be particularly preferred, for example the Syntex^{™} adjuvant emulsion (SAF-m, an emulsion consisting of squalene, Pluronlc L121 block polymer and Tween 80 in phosphate-buffered saline).

Other suitable agents for use as auxiliary adjuvants include live antigen-presenting cells (APCs). Particularly preferred are dendritic cells (DCs), which are preferably autologous and pre-loaded with antigen.

Alternatively, or in addition, Immune effector cells are used to promote a type 1 response, including cytotoxic T lymphocytes (CTLs). Again, such CTLs are preferably autologous and pre-targeted by exposure to DCs (and/or other APCs), or by *in vitro* targeting/priming techniques (including the *In vitro* manipulation of isolated CTLs by recombinant DNA techniques).

The auxiliary adjuvant(s) may be physically associated with the alkaloid of the invention: for example, the auxiliary adjuvant may be in admixture with the alkaloid. In embodiments where the auxiliary adjuvant comprises alum, the alkaloid (and any other auxiliary adjwant(s)) is preferably adsorbed onto the alum. Alternatively, the auxiliary adjuvant and alkaloid may be physically dissociated and merely co-administered (as herein defined), as further described below In the section entitled "Posology".

In another aspect, the invention provides a vaccine comprising the composition of the invention together with one or more antigen(s).

The invention finds application the manufacture of any vaccine, but may be particularly as a subunit vaccine, a conjugate vaccine, a DNA vaccine, a recombinant vaccine or a mucosal vaccine. The vaccine may be therapeutic or prophylactic.

Any suitable antigen or combination of antigens may be used in the vaccines of the invention, including for example nucleic add(s) which encode one or more antigenic protein(s); protein(s) or peptide(s); glycopratein(s); polysaccharide(s) and other carbohydrate(s)); fusion protein(s); lipid(s); glycolipid(s); peptide mimic(s) of polysaccharides; carbohydrate(s) and a protein(s) in admixture; carbohydrate-protein conjugate(s); cells or extracts thereof; dead or attenuated cells, or extracts thereof; tumour cells or extracts thereof; viral particles (e.g. attenuated viral particles or viral components) and allergen(s).

The antigen may comprises a bacterial, viral, fungal, protozoal or prion antigen. Other suitable antigens include neoantigens, tumour-associated antigens and self-antigen.

The antigen used In the vaccines of the invention may be dose-spared, since the adjuvant effect of the alkaloid(s) of the invention may permit sufficient immune stimulatory effect from relatively low amounts of antigen.

The vaccine may be administered by any suitable route, as explained In more detail in the section entitled "Posology" (below). Thus, the vaccine may be administered orally, topically, epicutaneously, intramuscularly, intradermally, subcutaneously, Intranasally, intravaginally, sublingually or *via* inhalation.

### Detailed Description of the Invention

### Definitions

Where used herein and unless specifically indicated otherwise, the following terms are intended to have the following meanings in addition to any broader (or narrower) meanings the terms might enjoy In the art:

As used herein, the term *alum* is intended to cover any aluminium compound, including aluminium salts (for example aluminium hydroxide, aluminium phosphate and mixtures thereof). Aluminum hydroxide for use as an adjuvant Is commercially available as Alhydrogel^{™}.

As used herein, the term *type 1 adjuvant* or *Th1 adjuvant* is Intended to define an adjuvant which stimulates a Th1 (type 1) response (or a response which is polarized or skewed towards a type 1 response, with a relatively weak Th2 (type 2) response). Such adjuvants include CpG adjuvants (as herein defined), MDP adjuvants (as herein defined), ISCOMS, some cytokines (including IL-12) and lipid A derivatives (including MPL).

As used herein, the term *type 2 adjuvant* or *Th2 adjuvant* is intended to define an adjuvant which stimulates a Th2 (type 2) response (or a response which is polarized or skewed towards a type 2 response, with a relatively weak Th1 (type 1) response). Such adjuvants include alum and submicron oil-in-water emulsion adjuvants (as herein defined, including MF59).

As used herein, the term *balanced adjuvant* is intended to define an adjuvant which stimulates both a Th1 (type 1) and a Th2 (type 2) response. Such adjuvants include saponin adjuvants (as herein defined), including QS-21 and certain experimental adjuvant systems (such as the alum/IL-12 adjuvant system described in Pollock *et a*/*.* (2003), *infra*).

As used herein, the term *adjuvant system* is intended to define an adjuvant composition comprising two or more different adjuvants. Exemplary adjuvant systems include SB-AS2 (SmithKline Beecham adjuvant system #2, an oil-in-water emulsion containing MPL and QS21) and SB-AS4 (SmithKline Beecham adjuvant system #4, which contains alum and MPL).

As used herein, the term *Th1-activating alkaloids* is intended to define an alkaloid (as hereinbefore defined) that can stimulate a type 1 immune response to an antigen when co-administered therewith at an appropriate dose. Thus, Th1-activating alkaloids can act as type 1 adjuvants. Preferred Th1-activating alkaloids are alkaloids that, when co-administered with antigen and alum, shift the balance of (or polarize) the Th2 alum-induced immune response from Th2 towards Th1 (so producing a balanced adjuvant activity profile). Particularly preferred are Th1-activating alkaloids which, when co-administered with alum and antigen, polarize the immune response such that the alum-induced Th2 response is augmented with a Th1 response.

As used herein, the terms *saponin* or *saponin adjuvant* is intended to define a family of glycosidic triterpenoid compounds which produce foam in aqueous solution and possess immune adjuvant activity. The term is also intended to cover natural and pharmaceutically acceptable salts and pharmaceutically acceptable derivatives as well as biologically active fragments thereof.

As used herein, the term CpG or *CpG adjuvant* is intended to define a family of nucleic acids (for example, oligonucleotides) which having at least one unmethylated CpG dinucleotide and which possess immune adjuvant activity.

As used herein, the term *submicron oil-in-water emulsion* or *submicron oil in-water emulsion adjuvant* is intended to cover a class of emulsions which comprise a metabolizable oil and an emulsifying agent, wherein the oil and the emulsifying agent are present in the form of an oil-in-water emulsion having oil droplets substantially all of which are less than 1 micron in diameter and which possess immune adjuvant activity.

As used herein, the term *MDP or MDP adjuvant* is intended to define derivatives of muramyl dipeptide suitable for use as adjuvants. An exemplary MDP adjuvant is N-acetylmuramyl-L-alanyl-D-isoglutaminyl-L-alanine-2-[1,2-dipalmitoyl-sn-glycero-3-3(hydroxyphosphoryloxy)]ethylamide (MTP-PE). This muramyl tripeptide has phospholipid tails that allow association of the hydrophobic portion of the molecule with a lipid environment while the muramyl peptide portion associates with the aqueous environment.

As used herein, the term *therapeutic vaccine* is intended to define a subclass of vaccines which have therapeutic (and not just prophylactic) properties. Such vaccines may have prophylactic activity in addition to therapeutic potential. They find application in the treatment of existing diseases, infections or conditions. They have become increasingly important as agents for the treatment of cancers, AIDS and malaria.

As used herein, the term *co-administration*, as used in the context of the administration of the various components of the compositions, vaccines etc. of the invention, is intended to cover the sequential, concurrent or separate administration of the referenced components. Concurrent administration therefore covers the case where the referenced components are physically mixed prior to administration. Sequential administration covers circumstances in which the referenced components are administered separately with some degree of temporal separation (typically from several minutes to several hours, although in some embodiments the administration of the co-administered components may be separated by a period of one or more days).

The term *neoantigen* is used herein to define any newly expressed antigenic determinant. Neoantigens may arise upon conformational change in a protein, as newly expressed determinants (especially on the surfaces of transformed or infected cells), as the result of complex formation of one or more molecules or as the result of cleavage of a molecule with a resultant display of new antigenic determinants. Thus, as used herein, the term neoantigen covers antigens expressed upon infection (e.g. viral infection, protozoal infection or bacterial infection), in prion-mediated diseases (e.g. BSE and CJD), an on cell transformation (cancer), in which latter case the neoantigen may be termed a tumour-associated antigen.

The term *tumour-associated antigen* is used herein to define an antigen present in transformed (malignant or tumourous) cells which is absent (or present in lower amounts or in a different cellular compartment) in normal cells of the type from which the tumour originated. Oncogenic viruses can also induce expression of tumour antigens, which are often host proteins induced by the virus.

The terms *polar* and *non-polar* are to be understood as relative terms which can be applied in the characterization of solvents to indicate the degree to which they have an electric dipole moment and so display hydrophilicity (polar) or hydrophobicity (non-polar). Such solvents can be used to extract polar and non-polar phytochemicals, respectively, and the terms *polar* and *non-polar,* as applied herein to alkaloids, phytochemicals or any other moieties, are to be interpreted accordingly.

The term *isolated* as applied to the alkaloids of the invention is used herein to indicate that the alkaloid exists in a physical milieu distinct from that in which it occurs in nature. For example, the isolated material may be substantially isolated (for example purified) with respect to the complex cellular milieu in which it naturally occurs. When the isolated material is purified, the absolute level of purity is not critical and those skilled in the art can readily determine appropriate levels of purity according to the use to which the material is to be put. Preferred, however, are purity levels of 90% w/w, 99% w/w or higher. In some circumstances, the isolated alkaloid forms part of a composition (for example a more or less crude extract containing many other substances) or buffer system, which may for example contain other components. In other circumstances, the isolated alkaloid may be purified to essential homogeneity, for example as determined spectrophotometrically, by NMR or by chromatography (for example GC-MS).

The terms *derivative* and *pharmaceutically acceptable derivative* as applied to the alkaloids of the invention define alkaloids which are obtained (or obtainable) by chemical derivatization of the parent alkaloids of the invention. The pharmaceutically acceptable derivatives are suitable for administration to or use in contact with the tissues of humans without undue toxicity, irritation or allergic response (i.e. commensurate with a reasonable benefit/risk ratio). Preferred derivatives are those obtained (or obtainable) by alkylation, esterification or acylation of the parent alkaloids of the invention. The derivatives may be immunostimulatory *perse,* or may be inactive until processed *in vivo.* In the latter case, the derivatives of the invention act as pro-drugs. Particularly preferred pro-drugs are ester derivatives which are esterified at one or more of the free hydroxyls and which are activated by hydrolysis *in vivo.* The pharmaceutically acceptable derivatives of the invention retain some or all of the immunostimulatory activity of the parent alkaloid. In some cases, the immunostimulatory activity is increased by derivatization. Derivatization may also augment other biological activities of the alkaloid, for example bioavailability and/or glycosidase inhibitory activity and/or glycosidase inhibitory profile. For example, derivatization may increase glycosidase inhibitory potency and/or specificity.

The term *pharmaceutically acceptable salt* as applied to the alkaloids of the invention defines any non-toxic organic or inorganic acid addition salt of the free base compounds which are suitable for use in contact with the tissues of humans and lower animals without undue toxicity, irritation, allergic response and which are commensurate with a reasonable benefit/risk ratio. Suitable pharmaceutically acceptable salts are well known in the art. Examples are the salts with inorganic acids (for example hydrochloric, hydrobromic, sulphuric and phosphoric acids), organic carboxylic acids (for example acetic, propionic, glycolic, lactic, pyruvic, malonic, succinic, fumaric, malic, tartaric, citric, ascorbic, maleic, hydroxymaleic, dihydroxymaleic, benzoic, phenylacetic, 4-aminobenzoic, 4-hydroxybenzoic, anthranilic, cinnamic, salicylic, 2-phenoxybenzoic, 2-acetoxybenzoic and mandelic acid) and organic sulfonic acids (for example methanesulfonic acid and p-toluenesulfonic acid). The alkaloid of the invention may also be converted into salts by reaction with an alkali metal halide, for example sodium chloride, sodium iodide or lithium iodide. Preferably, the alkaloids of the invention are converted into their salts by reaction with a stoichiometric amount of sodium chloride in the presence of a solvent such as acetone.

These salts and the free base compounds can exist in either a hydrated or a substantially anhydrous form. Crystalline forms of the compounds of the invention are also contemplated and in general the acid addition salts of the alkaloids of the invention are crystalline materials which are soluble in water and various hydrophilic organic solvents and which in comparison to their free base forms, demonstrate higher melting points and an increased solubility.

The present invention contemplates the use of all optical isomers, racemic forms and diastereomers of the alkaloids of the invention. Those skilled in the art will appreciate that, owing to the asymmetrically substituted carbon atoms present in the alkaloids of the invention, the alkaloids of the invention may exist and be synthesised and/or isolated in optically active and racemic forms. Thus, references to the alkaloids of the present invention encompass the alkaloids as a mixture of diastereomers, as individual diastereomers, as a mixture of enantiomers as well as in the form of individual enantiomers.

Therefore, the present invention contemplates all optical isomers and racemic forms thereof of the alkaloids of the invention, and unless indicated otherwise (e.g. by use of dash-wedge structural formulae) the compounds shown herein are intended to encompass all possible optical isomers of the compounds so depicted. In cases where the stereochemical form of the alkaloid is important for pharmaceutical utility, the invention contemplates use of an isolated eutomer.

### Alkaloids for Use According to the Invention

The alkaloid to be used according to the invention, stimulates a type 1 immune response to an antigen. Thus, the alkaloids for use according to the invention act as type 1 adjuvants. Preferred are alkaloids that, when co-administered with antigen and alum, shift the balance of (or polarize) the Th2 alum-induced Immune response from Th2 towards Th1 (so producing a balanced adjuvant activity profile). Particularly preferred are alkaloids which, when co-administered with alum and antigen, polarize the Immune response such that the alum-Induced Th2 response is augmented with a Th1 response.

Such alkaloids are herein referred to as *Th1-activating alkaloids,* and the Th1-activating alkaloid defined in the claims is used according to the invention.

Th1-activating alkaloids may be readily identified by screening assays designed to detect Th1 induction *in vitro*. Those skilled in the art will be aware of many suitable assays, such as those described herein and In Pollock et al. (2003) Immunology 108: 137-143, which is incorporated herein by reference (see In particular the section entitled "Materials and Methods" on page 13B). Other suitable assays can be readily selected or developed by those skilled In the art, who will readily be able to identify appropriate conditions for such assays, including *inter alia* the nature and number of the immune cells, the relative concentrations of alkaloid and cells, the duration of stimulation with alkaloid and the methods used to detect the induction of one or more cytokine(s).

In many embodiments of the invention, the alkaloid is isolated. However, in some embodiments the use of an isolated alkaloid is not required, and crude extracts suffice.

The alkaloids need not be naturally occurring, and may be synthetic analogues or derivatives of naturally occurring counterparts. Such analogues or derivatives are preferably pharmaceutically acceptable analogues, salts, isomers or derivatives as herein defined. However, preferred alkaloids are phytochemicals or derivatives or synthetic analogues thereof. Such phytochemicals may be isolated from natural sources or synthesised *in vitro*.

Preferred are alkaloids having a small molecular weight, since these may exhibit desirable pharmacokinetics. Thus, the alkaloid may have a molecular weight of 100 to 400 Daltons, preferably 150 to 300 Daltons and most preferably 200 to 250 Daltons.

The alkaloid has the formula: 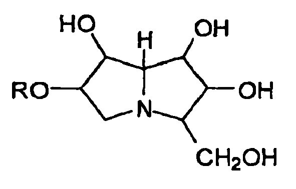 wherein R is selected from the group comprising hydrogen, straight or branched, unsubstituted or substituted, saturated or unsaturated acyl, alkyl (e.g. cycloalkyl), alkenyl, alkynyl and aryl groups, or a pharmaceutically acceptable salt or acyl derivative thereof.

Particularly preferred are alkaloids having the formula: 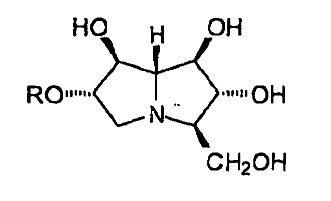 wherein R is selected from the group comprising hydrogen, straight or branched, unsubstituted or substituted, saturated or unsaturated acyl, alkyl (e.g. cyclcalkyl), alkenyl, alkynyl and aryl groups, or a pharmaceutically acceptable salt or derivative thereof.

In a particularly preferred embodiment the alkaloid is 1R,2R,3R,6S,7S,7aR)-3-(hydroxymethyl)-1,2,6,7-tetrahydroxypyrrolizidine (casuarine), wherein R is hydrogen and having the formula: 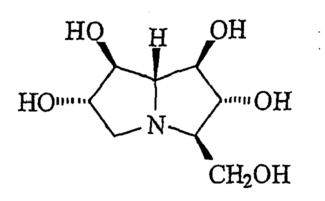 or a pharmaceutically acceptable salt or derivative thereof.

The alkaloid may also be a casuarine glycoside, or a pharmaceutically acceptable salt or derivative thereof. In such embodiments, the alkaloid is preferably casuarine-6-α-D-glucoside of the formula: 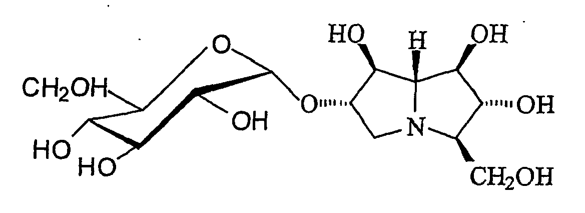 or a pharmaceutically acceptable salt or derivative thereof.

Other suitable alkaloids for use according to the invention are selected from:
(a) 3,7-*diepi*-casuarine;
(b) 7-*epi*-casuarine;
(c) 3,6,7-*triepi*-casuarine;
(d) 6,7-*diepi*-casuarine;
(e) 3-*epi*-casuarine;
(f) 3,7-*diepi*-casuarine-6-α-D-glucoside;
(g) 7-*epi*-casuarine-6-α-D-glucoside;
(h) 3,6,7-*triepi*-casuarine-6-α-D-glucoside;
(i) 6,7-*diepi*-casuarine-6-α-D-glucoside; and
(j) 3-*epi*-casuarine-6-α-D-glucoside,
or a pharmaceutically acceptable salt or derivative thereof.

Thus, particularly preferred are diastereomers of casuarine selected from 3,7-*diepi*-casuarine (14), 7-*epi-*casuarine (15), 3,6,7-*triepi*-casuarine (16), 6,7-*diepi*-casuarine (17) and 3-*epi*-casuarine (18), as well as pharmaceutically acceptable salts and derivatives thereof.

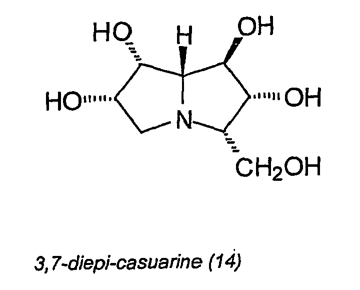

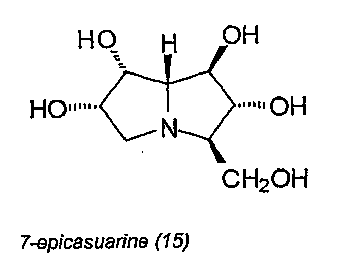

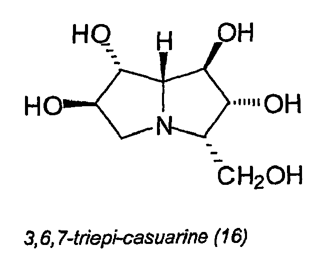

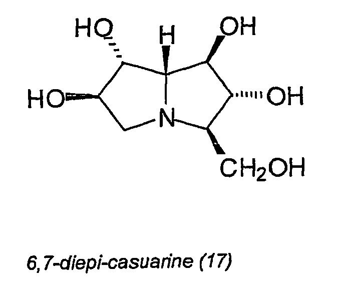

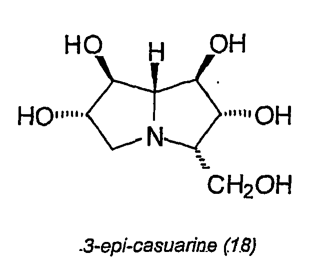

Other preferred diastereomers are selected from 3,7-*diepi*-casuarine-6-α-D-glucoside (19), 7-*epi*-casuarine-6-α-D-glucoside (20), 3,6,7-*triepi-*casuarine-6-α-D-glucoside (21), 6,7-*diepi*-casuarine-6-α-D-glucoside (22) and 3-*epi*-casuarine-6-α-D-glucoside (23), as well as pharmaceutically acceptable salts and derivatives thereof.

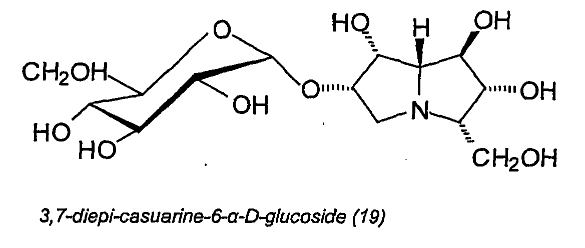

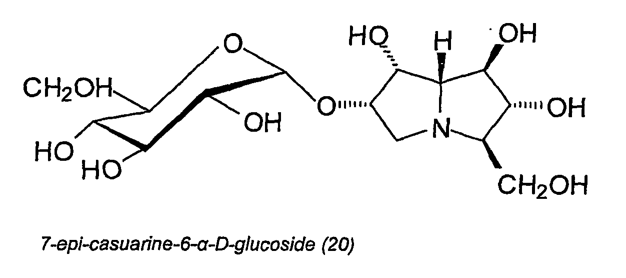

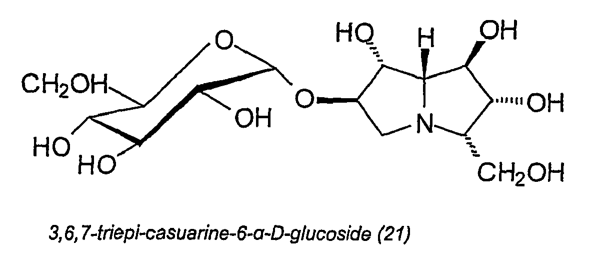

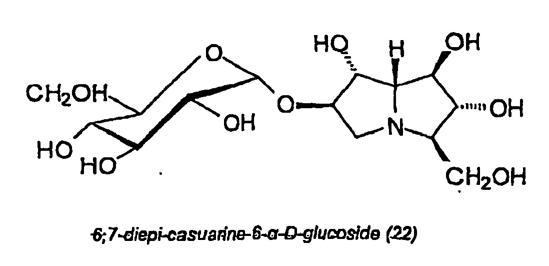

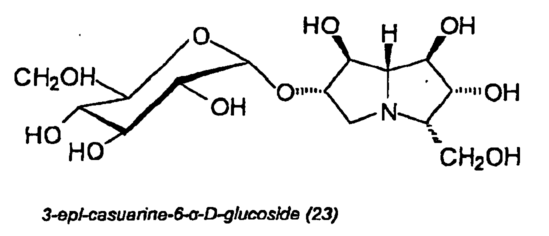

Other preferred diastereomers include 7a epimers selected from 3,7,7a-tr*iepi*-casuarine, 7,7a-*diepi*-casuarine, 3,6,7,7a-*tatraepi*-casuarine, 6,7,7a-tri*epi*-casuarine and 3,7a-di*epi*-casuarine, as well as pharmaceutically acceptable salts and derivatives thereof.

Particularly preferred are alkaloids selected from the table below:

| **COMPOUND** | **STRUCTURE** |
|---|---|
| casuarine (8) | 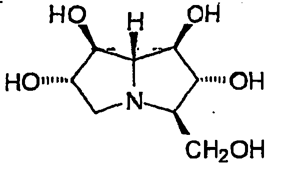 |
| casuarine-6-α-D-glucopyranose (9) | 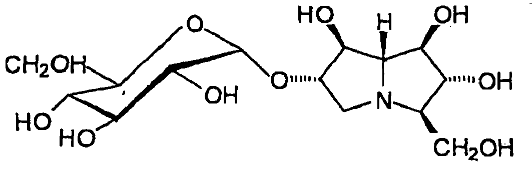 |
| 3,7-di*epi*-casuarine (10) | 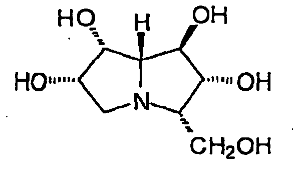 |
| 7-*epi*-casuarine (11) | 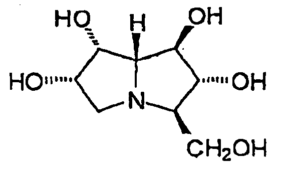 |
| 3-*epi*-casuarine (14) | 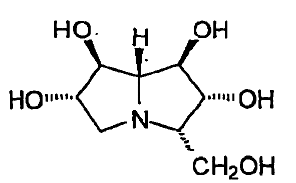 |

(or stereochemical variants thereof).

### Biological Activities of the Alkaloids of the Invention

The alkaloids of the invention stimulate a type 1 immune response to an antigen. Thus, the alkaloids of the invention may act as type 1 adjuvants. They may therefore be employed in conjunction with various auxiliary adjuvants to shift the balance of (or polarize) the Immune response towards a type 1 profile. Thus, the alkaloids of the invention may be used in conjunction with a type 2 adjuvant (such as alum) to shift the immune response from Th2 towards Th1, so producing a balanced adjuvant activity profile. The alkaloids may therefore polarize the immune response such that an alum-induced Th2 response is augmented with a Th1 response.

The type 1 immune response exhibited by the alkaloids of the invention may be a dominant type 1 response. A dominant type 1 response is one that can augment a parallel type 2 response (for example, one that has arisen endogenously or been stimulated by a co-administered auxiliary type 2 or balanced adjuvant) with a type 1 component. A dominant type 1 response permits the production of balanced adjuvant systems by co-administration of the alkaloid of the invention with one or more adjuvants having type 2 activity, as described herein. Particularly preferred are alkaloids which exhibit a dominant type 1 response such that, on co-administration with alum, the alum-Induced Th2 response is augmented with an alkaloid-induced type 1 response.

Without wishing to be bound by any theory, it is thought that the polarizing adjuvant activity of the alkaloids of the invention arises from the stimulation of the expression of one or more Th1 cytokines and/or the suppression of one or more Th2 cytokines *in vivo.* Thus, the alkaloids of the invention may stimulate the expression of one or more Th1 cytokines *in vivo*. Preferred are alkaloids that stimulate IL 12 and/or IL-2 *in vivo* or *in vitro* (for example in lymphocytes and/or dendritic cells). Particularly preferred are Th1-activating alkaloids that stimulate the production of IL-2 in dendtritic cells *in vitro*.

IL-2 is a Th1 cytokine involved in mediating type-1 responses. It appears to be involved not only in T cell activation but also in the activation of *inter alia* NK cells, so functioning to regulate and link innate and adaptive immunity. Thus, alkaloid-induced expression of IL-2 in dendritic cells may directly potentiate a Th1 response and so increase the Th1:Th2 response ratio. The alkaloid-induced expression of IL-2 may also indirectly potentiate a Th1 response (and so increase the Th1:Th2 response ratio) by stimulating the activity of endogenous dendritic cells, which cells then trigger responses by other classes of lymphocytes (CTL, B, NK, and NKT cells) and also elicit T cell memory (a critical goal of vaccination).

IL-12 is the primary mediator of type-1 immunity (the Th1 response). It induces NK cells to produce IFN-γ as part of the innate immune response and promotes the expansion of CD4 Th1 cells and cytotoxic CD8 cells which produce IFN-γ. It therefore increases T-cell invasion of tumours as well as the susceptibility of tumour cells to T-cell invasion. Thus, an alkaloid-induced expression of IL-12 in lymphocytes (e.g. in dendritic cells and/or macrophages) may directly potentiate a Th1 response and so increase the Th1:Th2 response ratio.

The alkaloids of the invention may also suppress the expression of one or more Th2 cytokines (e.g. IL-5), so increasing the Th1:Th2 response ratio.

The alkaloids of the invention may also be glycosidase inhibitors. Particularly preferred are alkaloids which exhibit specificity of glycosidase inhibition, for example Glucosidase I rather than mannosidase. Such preferred alkaloids can therefore be quite different in their glycosidase inhibitory profile to swainsonine and its analogues, since the latter are potent and specific inhibitors of mannosidase. Any such glycosidase inhibition may be involved in mediating the stimulation of the type 1 response, or may be purely incidental to the polarizing adjuvant activity. However, without wishing to be bound by any theory, it is thought that such activity may serve as an index or marker of type 1 adjuvant activity in the Th1-activating alkaloids of the invention.

### Vaccine targets

The vaccines of the invention may be used in the treatment or prophylaxis of a wide range of diseases and disorders, as described below.
- *Viral targets* include diseases and disorders in which any of the following viruses (or virus classes) are implicated: *Retroviridae* (e.g. the human immunodeficiency viruses, including HIV-1); *Picornaviridae* (e.g. polio viruses, hepatitis A virus; enteroviruses, human Coxsackie viruses, rhinoviruses, echoviruses); *Calciviridae* (e.g. strains that cause gastroenteritis); *Togaviridae* (e.g. equine encephalitis viruses, rubella viruses); *Flaviridae* (e.g. dengue viruses, encephalitis viruses, yellow fever viruses); *Coronoviridae* (e.g. coronaviruses); Rhabdoviradae (e.g. vesicular stomatitis viruses, rabies viruses); *Filoviridae* (e.g. ebola viruses); *Paramyxoviridae* (e.g. parainfluenza viruses, mumps virus, measles virus, respiratory syncytial virus); *Orthomyxoviridae* (e.g. influenza viruses); *Bungaviridae* (e.g. Hantaan viruses, bunga viruses, phleboviruses and Nairo viruses); *Arena viridae* (hemorrhagic fever viruses); *Reoviridae* (e.g. reoviruses, orbiviurses and rotaviruses); *Bimaviridae*; *Hepadnaviridae* (Hepatitis B virus); *Parvoviridae* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); and *Iridoviridae* (e.g. African swine fever virus); and unclassified viruses (e.g. the etiological agents of Spongiform encephalopathies, the agent of delta hepatitis (thought to be a defective satellite of hepatitis B virus), the HCV virus (causing non-A, non-B hepatitis); Norwalk and related viruses, and astroviruses). Of the foregeoing, particularly preferred are HIV, Hepatitis A, Hepatitis B, Hepatitis C, rabies virus, poliovirus, influenza virus, meningitis virus, measles virus, mumps virus, rubella, pertussis, encephalitis virus, papilloma virus, yellow fever virus, respiratory syncytial virus, parvovirus, chikungunya virus, haemorrhagic fever viruses and Herpes viruses, particularly, varicella, cytomegalovirus and Epstein-Barr virus. In such embodiments the antigen(s) selected for use in the vaccine are derived from (or designed by reference to) those antigens present in the naturally-occurring virus (or expressed/induced thereby during infection).
- *Bacterial targets* include both Gram-negative and Gram-positive bacteria. Examples of bacteria which may be targeted by the vaccines of the invention include but are not limited to: *Helicobacter pylori, Borelia burgdorferi, Legionella pneumophilia, Mycobacterium* spp (e.g. *M. tuberculosis, M. leprae, M. avium, M. intracellulare, M. kansaii* and *M. gordonae*), *Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus viridans, Streptococcus faecalis, Streptococcus bovis,* any of the anaerobic species of the genus *Streptococcus, Streptococcus pneumoniae, Campylobacterspp., Enterococcus* spp., *Haemophilus influenzae, Bacillus anthracis, Corynebacterium* spp. (including *C. diphtheriae*), *Erysipelothrix rhusiopathiae, Clostridium perfringens, Clostridium tetani, Enterobacter aerogenes, Klebsiella* spp (including *K. pneumoniae*), *Pasturella multocida, Bacteroides* spp., *Fusobacterium nucleatum, Streptobacillus monilijormis, Treponema pallidium, Treponema pertenue, Leptospira* spp., *Rickettsia* spp. and *Actinomyces* spp. (including A. *israelii*). In such embodiments the antigen(s) selected for use in the vaccine are derived from (or designed by reference to) those antigens present in the naturally-occurring bacterium (or expressed/induced thereby during infection).
- *Fungal targets* include *Cryptococcus neoformans, Histoplasma capsulatum, Coccidioides immitis, Blastomyces dermatitidis, Chlamydia trachomatis* and *Candida albicans.* In such embodiments the antigen(s) selected for use in the vaccine are derived from (or designed by reference to) those antigens present in the naturally-occurring fungus (or expressed/induced thereby during infection).
- *Protozoal targets* include *Plasmodium* spp. (including *Plasmodium falciparum, Plasmodium malariae, Plasmodium ovale* and *Plasmodium vivax*), *Toxoplasma* spp. (including *T. gondii* and *T. cruzii*) and *Leishmania* spp.
- *Cancers and proliferative disorders* including solid tissue cancers and those of the blood and lymphatic systems (including Hodgkin's Disease, leukemias, lymphomas, multiple myeloma, and Waldenström's disease), melanomas (including melanoma of the eye), adenomas, sarcomas, carcinomas of solid tissues, melanoma, cancers of the lung, thyroid, salivary gland, leg, tongue, lip, bile duct, pelvis, mediastinum, urethra, Kaposi's Sarcoma (e.g. when associated with AIDS); skin cancers (including malignant melanoma), cancers of the digestive tract (including head and neck cancers, oesophageal cancer, stomach cancer, cancer of the pancreas, liver cancer, colon and rectal cancer, anal cancer), cancers of the genital and urinary systems (including kidney cancer, bladder cancer, testis cancer, prostate cancer), cancers in women (including breast cancer, cervico-uterine cancer, ovarian cancer, gynecological cancers and choriocarcinoma) as well as in brain, bone carcinoid, nasopharyngeal, retroperitoneal, thyroid, soft tissue tumours and cancers of unknown primary site. In such embodiments the antigen(s) selected for use in the vaccine are the cognate neoantigen(s) or tumour-associated antigen(s) present in the malignant cells and/or tissues.
- *Allergic disorders* include atopic allergy, allergic rhinitis, allergic conjunctivitis, atopic dermatitis, hypereosinophilia, irritable bowel syndrome, allergen-induced migraine, bacterial allergy, bronchial allergy (asthma), contact allergy (dermatitis), delayed allergy, pollen allergy (hay fever), drug allergy, sting allergy, bite allergy, gastrointestinal or food allergy (including that associated with inflammatory bowel disease, including ulcerative colitis and Crohn's disease) and physical allergy. Physical allergies include cold allergy (cold urticaria or angioedema), heat allergy (cholinergic urticaria) and photosensitivity. In such embodiments the antigen(s) selected for use in the vaccine are derived from (or designed by reference to) those antigens present in the cognate allergen, including pollens, insect venoms, animal dander dust, fungal spores and drugs (e.g. penicillin) and proteins specific to the following genera: *Canis, Dermatophagoides, Felis, Ambrosia, Lolium, Cryptomeria, Alder*; *Alnus*; *Betula, Quercus, Festuca* and *Bromus.*
- *Other targets* include metazoan parasites or pathogens, such as helminths (e.g. *Schistosoma* spp.).

Thus, it can be seen that the compositions and vaccines of the invention find application in the treatment or prophylaxis of various infections, including bacterial, viral, fungal, protozoan and metazoan infections. For example, the vaccines may be used in the treatment or prophylaxis of infection with respiratory syncytial virus (RSV), Epstein-Barr, hepatitis B virus (HBV), hepatitis C virus (HCV), herpes simplex type 1 and 2, herpes genitalis, herpes keratitis, herpes encephalitis, herpes zoster, human immunodeficiency virus (HIV), influenza A virus, hantann virus (hemorrhagic fever), human papilloma virus (HPV), tuberculosis, leprosy and measles. Particularly preferred is the treatment or prophylaxis of infections in which the pathogen occupies an intracellular compartment or causes the expression of neoantigens by host cells, including HIV/AIDS, leishmania, influenza, tuberculosis and malaria.

In general, the target of the vaccines of the invention will be reflected in the nature of the antigen(s) selected for use in the vaccine formulation, and those skilled in the art will be able to select, design or derive the most appropriate antigen(s) from the causative agents (as listed above).

### Veterinary applications

The invention not only finds application in the field of human therapy and prophylaxis, but also in the treatment and/or prophylaxis of infections in any non-human animal. Such veterinary applications are described in more detail below.
- *Domesticated animals.* The development of vaccines for prophylactic or therapeutic use with domesticated animals is of great importance, not only because the market for such vaccines for use with domestic pets is extremely large but also because the close proximity of animal and human in such situations leads to an increased risk of pathogen transmission from animal to human. Such "cross-species jumping" has been responsible for the evolution of many important human pathogens, including measles, HIV, SARS and dengue fever. The influenza A virus jumped from birds into humans to produce devastating pandemics in 1918, 1957 and 1968. Thus, the invention finds application in the treatment or prophylaxis of domesticated animals. Domesticated animals include pets (such as dogs, cats, mice, rats, gerbils, hamsters, monkeys, ferrets, fish, pigeons, parrots and other birds) as well as working animals such as horses, dogs, donkeys and goats.
- *Cattle.* Infection of cattle and livestock can produce severe economic losses, as shown by the recent outbreaks of foot and mouth in the United Kingdom. Thus, vaccines for use with cattle and livestock (including cows, horses, donkeys, pigs, sheep, and goats) are extremely important.
- *Birds.* Modem farming practice often houses birds in large numbers in very close proximity under closed conditions. Such conditions foster the rapid spread of infectious disease, and the control of infection in birds is a major concern. Also, hatchlings are particularly vulnerable to infection. Particularly preferred is the treatment of birds selected from chicken, pigeon, turkey, duck, geese, pheasant and quail.
- *Fish and other aquatic animals.* The high density of aquatic animals in hatchery tanks, fish farms or other types of marine or freshwater farming enclosures puts the aquaculture industry at particular risk from infections and infestations. Viral, bacterial and parasitic diseases pose a serious problem for the aquaculture industry. Thus, the Invention finds application in the treatment or prophylaxis of fish and other aquatic animals, including fish and shellfish (including clams, lobster, shrimp, crab and oysters). Particularly preferred is the treatment or prophylaxis of bony or cartilaginous fish, including salmonids, carp, catfish, yellowtail, seabream, and seabass. Particularly preferred is the treatment of trout, salmon and char.

### Formulation

The compositions and vaccines of the invention comprise the alkaloid of the invention, optionally together with one or more auxiliary adjuvants and/or a pharmaceutically acceptable excipient.

The alkaloid of the invention may take any form. It may be synthetic, purified or isolated from natural sources (for example from *Casuarina equisetifolia* or *Eugenia jambolana*), using techniques described in the art (and referenced *infra*). When isolated from a natural source, the alkaloid of the invention may be purified.

In embodiments where the alkaloid of the invention is formulated together with a pharmaceutically acceptable excipient, any suitable excipient may be used, including for example inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc.

The pharmaceutical compositions may take any suitable form, and include for example tablets, elixirs, capsules, solutions, suspensions, powders, granules and aerosols.

The pharmaceutical composition may take the form of a kit of parts, which kit may comprise the composition of the invention together with instructions for use and/or a plurality of different components in unit dosage form.

Tablets for oral use may include the alkaloid of the invention, either alone or together with other plant material associated with the botanical source(s). The tablets may contain the alkaloid of the invention mixed with pharmaceutically acceptable excipients, such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the alkaloid of the invention is mixed with a solid diluent, and soft gelatin capsules wherein the active ingredient is mixed with water or an oil such as peanut oil, liquid paraffin or olive oil.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

For intramuscular, intraperitoneal, subcutaneous and intravenous use, the compounds of the invention will generally be provided in sterile aqueous solutions or suspensions, buffered to an appropriate pH and isotonicity.

Suitable aqueous vehicles include Ringer's solution and isotonic sodium chloride. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate.

The compounds of the invention may also be presented as liposome formulations.

For oral administration the alkaloid of the invention can be formulated into solid or liquid preparations such as capsules, pills, tablets, troches, lozenges, melts, powders, granules, solutions, suspensions, dispersions or emulsions (which solutions, suspensions dispersions or emulsions may be aqueous or non-aqueous). The solid unit dosage forms can be a capsule which can be of the ordinary hard- or soft-shelled gelatin type containing, for example, surfactants, lubricants, and inert fillers such as lactose, sucrose, calcium phosphate, and cornstarch.

In another embodiment, the alkaloids of the invention are tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient.

Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent or emulsifying agent.

The alkaloids of the invention may also be administered parenterally, that is, subcutaneously, intravenously, intramuscularly, or interperitoneally.

In such embodiments, the alkaloid is provided as injectable doses in a physiologically acceptable diluent together with a pharmaceutical carrier (which can be a sterile liquid or mixture of liquids). Suitable liquids include water, saline, aqueous dextrose and related sugar solutions, an alcohol (such as ethanol, isopropanol, or hexadecyl alcohol), glycols (such as propylene glycol or polyethylene glycol), glycerol ketals (such as 2,2-dimethyl-1,3-dioxolane-4-methanol), ethers (such as poly(ethylene-glycol) 400), an oil, a fatty acid, a fatty acid ester or glyceride, or an acetylated fatty acid glyceride with or without the addition of a pharmaceutically acceptable surfactant (such as a soap or a detergent), suspending agent (such as pectin, carhomers, methylcellulose, hydroxypropylmethylcellulose, or carboxymethylcellulose), or emulsifying agent and other pharmaceutically adjuvants. Suitable oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil.

Suitable fatty acids include oleic acid, stearic acid, and isostearic acid. Suitable fatty acid esters are, for example, ethyl oleate and isopropyl myristate.

Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulphonates, alkyl, olefin, ether, and monoglyceride sulphates, and sulphosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures.

The parenteral compositions of this invention will typically contain from about 0.5 to about 25% by weight of the alkaloid of the invention in solution. Preservatives and buffers may also be used. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The alkaloids of-the invention may also be administered topically, and when done so the carrier may suitably comprise a solution, ointment or gel base. The base, for example, may comprise one or more of the following: petrolatum, lanolin, polyethylene glycols, bee wax, mineral oil, diluents such as water and alcohol, and emulsifiers and stabilizers. Topical formulations may contain a concentration of the alkaloid from about 0.1 to about 10% w/v (weight per unit volume).

When used adjunctively, the alkaloids of the invention may be formulated for use with one or more other drug(s). In particular, the alkaloids of the invention may be used in combination with antitumor agents, antimicrobial agents, anti-inflammatories, antiproliferative agents and/or other immunostimulatory agents. For example, the alkaloids of the invention may be used with anti-viral and/or anti-proliferative agents such as cytokines, including interleukins-2 and 12, interferons and inducers thereof, tumor necrosis factor (TNF) and/or transforming growth factor (TGF), as well as with myelosuppressive agents and/or chemotherapeutic agents (such as doxorubicin, 5-fluorouracil, cyclophosphamide and methotrexate), isoniazid (e.g. in the prevention or treatment of peripheral neuropathy) and with analgesics (e.g. NSAIDs) for the prevention and treatment of gastroduodenal ulcers.

Thus, adjunctive use may be reflected in a specific unit dosage designed to be compatible (or to synergize) with the other drug(s), or in formulations in which the alkaloid is admixed with one or more antitumor agents, antimicrobial agents and/or antiinflammatories (or else physically associated with the other drug(s) within a single unit dose). Adjunctive uses may also be reflected in the composition of the pharmaceutical kits of the invention, in which the alkaloid of the invention is co-packaged (e.g. as part of an array of unit doses) with the antitumor agents, antimicrobial agents and/or antiinflammatories. Adjunctive use may also be reflected in information and/or instructions relating to the co-administration of the alkaloid with antitumor agents, antimicrobial agents and/or antiinflammatories.

### Posology

The amount of the alkaloid administered can vary widely according to the particular dosage unit employed, the period of treatment, the age, weight, kind of adjunctive treatment (if any), and sex of the patient treated, the nature and extent of the disorder treated, and the nature of the antigen, alkaloid and any auxiliary adjuvant(s) administered.

As explained earlier, the amount of alkaloid used in the vaccines of the invention (particularly the relative amount used when present with auxiliary adjuvant(s)) may be selected according to the balance of the Th1:Th2 response desired: the Th1:Th2 balance may be rationally preselected according to the nature of the ultimate vaccine target by titration of the Th1 response contributed by the alkaloid of the invention against the Th1 and/or Th2 response contributed by the auxiliary adjuvant(s). In this way, balanced adjuvants with a preselected Th1:Th2 ratio falling anywhere within a wide spectrum of {type 1 ↔ type 2) activities may be produced.

Moreover, the alkaloids of the invention can be used in conjunction with other agents known to be useful in the treatment of diseases, disorders or infections where immunostimulation is indicated (as described *infra*) and in such embodiments the dose may be adjusted accordingly.

The alkaloids of the present invention can be administered by oral or parenteral routes, including intravenous, intramuscular, intraperitoneal, subcutaneous, transdermal, mucosal, airway (aerosol), rectal, vaginal and topical (including buccal and sublingual) administration. Preferred routes of administration of the vaccines of the invention are subcutaneous or transdermal routes.

The vaccines are preferably prepared and administered in dose units. Liquid dose units are vials or ampoules for injection or other parenteral administration. Solid dose units are tablets, capsules and suppositories. For treatment of a patient, depending on activity of the compound, manner of administration, purpose of the immunization (i.e., prophylactic or therapeutic), nature and severity of the disorder, age and body weight of the patient, different doses may be necessary. The administration of a given dose can be carried out both by single administration in the form of an individual dose unit or else several smaller dose units. Multiple administration of doses at specific intervals of weeks or months apart is usual for boosting the antigen-specific responses.

The alkaloids of the invention may be administered concurrently, separately or sequentially with administration of the other vaccine components (including any auxiliary adjuvants, such as alum).

Thus, in some embodiments, the alkaloids of the invention may be present in admixture with other vaccine component(s), or else co-packaged (e.g. as part of an array of unit doses) with the other vaccine components with which it is to be used as adjuvant. In yet other embodiments, the use of the alkaloids of the invention as adjuvant is simply reflected in the content of the information and/or instructions co-packaged with the vaccine components and relating to the vaccination procedure, vaccine formulation and/or posology.

Where an auxiliary adjuvant is used, the alkaloid and auxiliary adjuvant may be co-administered (as defined herein), i.e. simultaneously or sequentially. When the adjuvants are administered simultaneously they can be administered in the same or separate formulations, and in the latter case at the same or separate sites, but may be administered at the same time. The adjuvants may be administered sequentially, when the administration of the at least two adjuvants is temporally separated. The separation in time between the administration of the two adjuvants may be a matter of minutes or it may be longer. The separation in time is less than 14 days, and more preferably less than 7 days, and most preferably less than 1 day. The separation in time may also be with one adjuvant at prime and one at boost, or one at prime and the combination at boost, or the combination of one at prime and one at boost.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Example 1: Effect of 3,7-diepi-casuarine (14) on alum-induced Th1/Th2 responses

### Adjuvant preparation

Alhydrogel (alum; purchased from Superfos BioSector a/s, Vedbaek, Denmark) was mixed with a predetermined quantity of highly purified ovalbumin (OVA; Worthington Biochemical Corporation, Lakewood, NJ) with or without 3,7-die*pi*-casuarine.

### Mice and inoculations

All mice (BALB/c) were immunized subcutaneously into the foodpad with 100µg OVA in 50µl phosphate-buffered saline (PBS) mixed with alum and either PBS (control) or 3,7-*diepi*-casuarine (50µg in PBS). Boosting inoculations were performed in the same fashion 2 weeks later, into the contralateral footpad.

### Determination of plasma antibody titres

Blood samples (tail bleeds) were taken 3 weeks after the primary inoculation. Enzyme-linked immunosorbent assays (ELISA) were performed as described in Brewer et al. (1999) J Immunol 163:6448-54 to detect OVA-specific IgG1 and IgG2a in plasma. Results were expressed as end-point dilutions where the end-point is determined as the final plasma dilution, which yields a higher absorbance than a negative control plasma sample, included in the assay.

### Isolation of Spleen cells and culture of spleen cells

The mouse spleen was removed aseptically and placed in a sterile petri dish containing 5mls of complete medium (RPMI, 1% L-Glutamine, 1% Penicillin/Streptomycin and 10% foetal calf serum). Cells suspensions were prepared by using the end of a syringe and grinding the spleen through a wire mesh. The cell suspension was then centrifuged at 1000rpm for 5 minutes. To remove the erythrocytes, the cell pellet was resuspended in Boyle's solution (Tris 0.17M & Ammonium Chloride 0.16M) and centrifuged again for 5 minutes. The pellet was then washed in medium a further two times, then resuspended in 3ml medium. A cell count was then carried out. Stimulation assays were performed in triplicate in U-bottomed 96-well plates (Corning Costar, NY) with OVA (at 5µg/ml, 100µg/ml and 500 µg/ml).

### Cytokine assays

Murine interferon-γ (IFN-γ) was assayed by ELISA using paired antibodies according to the manufacturer's instructions.

### Results

As described in Brewer ef al. (1999) J Immunol 163:6448=54, inoculation of BALB/e mice with highly purified ovalbumin prepared in alum produced high titres of antigen-specific IgG1 (Fig. 1a) and negligible amounts of IgG2a (Fig. 1b), a phenotype typical of a polarized Th2 response. The presence of 3,7-di*epi*-casuarine in admixture with the alum/OVA (MNLP24) resulted in a pronounced shift towards a Th1 response with a significant increase in IgG2a.

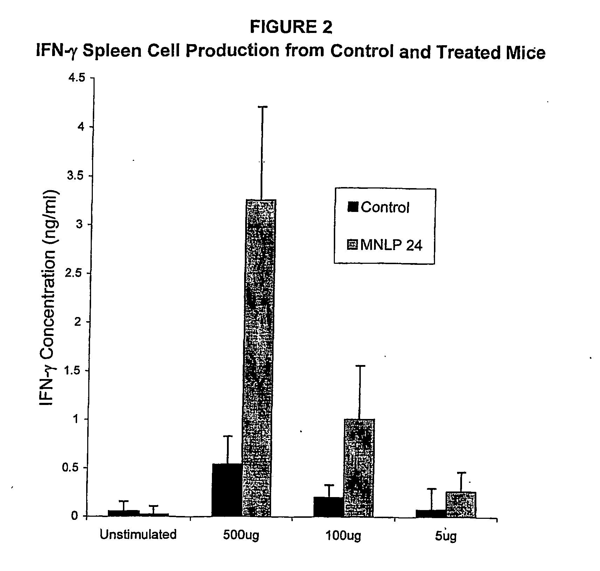

When spleen cells were re-stimulated with antigen *in vitro,* a significantly greater amount of IFN-γ was produced in cells derived from mice inoculated with alum/OVA in admixture with 3,7-di*epi*-casuarine (MNLP 24) as compared with those Inoculated with alum/OVA alone (control) (Fig. 2).

The results demonstrate that the Th2 profile of alum (which has been a major obstacle to its widespread use in modern vaccines) can be augmented with a Th1 component by co-administration of the Th1-activating alkaloid 3,7-di*epi*-casuarine. This opens up the possibility of tailoring the Th1:Th2 balance of the alum-induced immune response by the rational application of Th1-activating alkaloids (such as 3,7-di*epi*-casuarine).

### Comparative Example 2: Effect of exogenous rIL-12 on alum-induced Th1/Th2 responses

The experiment described in Example 1 (above) was repeated, but with the 3,7-di*epi*-casuarine replaced with 1µg of rIL-12 (R&D Systems, Oxford, UK). Proliferative response assays of IFN-γ were performed on lymph node cultures rather than spleen cells (as described in Pollock et al. (2003) Immunology108:137-143).

The results showed that rIL-12 has a comparable effect to 3,7-di*epi*-casuarine on the alum-induced Th1/Th2 response, producing a similar Th1-augmented Th2 response (see Figures 1 and 2, Pollock et al. (2003) Immunology108:137-143).

### Example 3: Adjuvant activity of 3,7-diepi-casuarine (MNLP 24) with an optimum dose of influenza vaccine

### Experimental design

Three groups of 6 female Balb/c mice were vaccinated intramuscularly (i.m.) with a mixture of the influenza vaccine together with 3,7-di*epi*-casuarine on days 0 and 14. Each group received a different dose of 3,7-di*epi-*casuarine. Group B received a low dose, group C a medium dose and group D a high dose (20, 50 and 100µg respectively). A fourth control group (group A) received only the influenza vaccine. Immunomodulatory activity was assessed by the determination of influenza-specific antibody responses in serum obtained at day 28.

### Materials and methods

*Mice*: 34 female, SPF-bred, BALB/c mice were obtained from a colony maintained under SPF-conditions at Charles River Deutschland, Sulzfeld, Germany. Twenty-four animals were allocated to the various groups by computer randomization. Mice were housed in type 2 macrolon cages in the same room throughout the study period at a temperature of 20.7-24.6°C and 30-70% humidity. The animal room was ventilated with at least 10 air changes per hour. The lighting was artificial by fluorescent tubes, time switch controlled at a sequence of 12 hours light, 12 hours dark (lights on from 7.00 a.m. to 7.00 p.m.). Feed and drinking water were provided ad *libitum.*

*Water for injection* was supplied by NPBI (the Netherlands), batch number 03G0472, expiry date June 2006, CBS number 42650, stored at ambient room temperature.

*Influenza vaccine* (Solvay Pharmaceuticals B.V, Weesp, the Netherlands) is a monovalent subunit vaccine containing haemaglutinin (HA) of the A/Panama strain. Concentration of stock was 430 µg HA/ml, batch number HPR34, stored at 2-10ºC.

*Stock 3,7-diepi-casuarine* (5.8 mg) was diluted in 2.0 ml water for injection (concentration 100 µg/35 µl) and 250 µl aliquots were prepared and stored.

Control group A received only influenza vaccine (105 µl influenza vaccine stock mixed with 245 µl water for injection). Group B received 49 µl from the stock 3,7-di*epi*-casuarine mixed with 105 µl influenza vaccine stock and 196 µl water for injection. Group C received 122.5 µl from the stock 3,7-di*epi*-casuarine mixed with 105 µl influenza vaccine stock and 122.5 µl water for injection. Group D received 245 µl from the stock 3,7-di*epi-*casuarine mixed with 105 µl influenza vaccine stock.

All solutions were prepared fresh, just prior to use.

Control group A received two i.m. injections in the thigh muscle of the left and right hind leg (25 µl per paw) with vaccine at days 0 and 14. Groups B-D were injected i.m. in the thigh muscle of the left and right hind leg (25 µl per paw) with vaccine and 3,7-di*epi*-casuarine.

### Determination of influenza vaccine-specific IgG, IgG1 and IgG2a

IgG, IgG1 (Th2-mediated) and IgG2a (Th1-mediated) antibodies against vaccine antigen were determined in the serum samples obtained at days 0 and 28 using sandwich ELISA. Flat bottom plates (NUNC Immuno Plate, Roskilde, Denmark) were coated with 100 µl (5 µg protein/ml), dissolved in carbonate buffer (pH 9.6). After washing three times (0.5% Tween-20 solution), the plates were blocked by adding 100 µl/well PBS containing 1% bovine serum albumin and 0.02% Tween 20 (PBS/Tween 0.02%/BSA 1%). After 1 h incubation at 37°C, the plates were washed and serial dilutions of test-serum (in PBS/Tween 0.02%/BSA1%) were incubated in duplicate (1 h, 37°C). Starting dilutions were 1:400. After a second washing step, HRP-conjugated antibody was added (30 min, 37°C); rat anti-mouse IgG, IgG1 or IgG2a (Zymed) diluted in PBS/BSA/Tween (1:1000). After a washing step, TMB-substrate solution (3,3',5,5'-tetramethylbenzidine) was added (20 min, RT), subsequently the reaction was stopped with 2N H₂SO₄. Optical density (OD) were measured at 450 nm using a Bio Rad microplate reader 3550 (Bio Rad Laboratories, Richmond, CA). Based upon a standard curve obtained with a reference (pooled) serum sample, containing an arbitrary number of units of specific antibodies per ml (AU/ml), concentrations of influenza-specific IgG, IgG1 and IgG2a in serum was calculated. Reference serum was prepared by mixing equal volumes of each individual serum sample obtained at day 28 from group A and was incorporated on each individual ELISA plate (starting dilution 1:50, serially diluted 11 times).

Statistical evaluation of the data was performed by analysis of (co)-variance followed by Dunnett's multiple comparison tests. In case of inhomogeneity of variance, the data were log transformed (in case of statistical analysis on specific IgG1 data). Probability values of p<0.05 were considered significant.

### Results

A good influenza vaccine-specific-IgG total and IgG1 antibody response was observed in all groups. The observed IgG2a response was much less pronounced in all test groups. Two values from one of the control animals was identified at the 99% confidence level as outliers and so excluded.

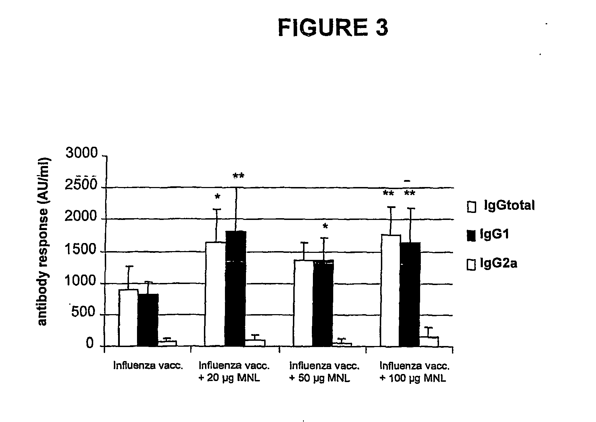

Statistically significant differences were observed between the control group A and all treatment groups B-D (see Figure 3, in which influenza vaccine-specific IgGtot, IgG1, and IgG2a serum antibody responses in animals vaccinated with influenza alone or in combination with 20, 50 or 100 µg 3,7-di*epi*-casuarine (labelled "MNL" on the X-axis) as measured by ELISA - the bars represent group means ± SD. * P<0.05, ** P<0.01 versus the control group vaccinated with influenza vaccine alone). No dose response was observed. A statistically significant increased specific IgG1 response was observed in the groups treated with 20 (P<0.01), 50 (P<0.05) and 100 µg 3,7-di*epi*-casuarine (P<0.01), respectively.

Compared to the control group A, a statistically significant increased IgGtotal response was observed in the groups treated with 20 (P<0.05) or 100 ug 3,7-di*epi*-casuarine (P<0.01), respectively. No differences in specific IgG2a responses were observed.

The results showed that 3,7-di*epi*-casuarine exhibits adjuvant activity even in vaccine formulations containing an optimum dose of influenza antigen: a clear specific IgGtotal and IgG1 response (Th2-mediated) was observed. The statistically significant increase in specific IgGtotal and IgG1 observed in the treatment groups B-D clearly shows the adjuvant activity of 3,7-di*epi*-casuarine. No skewing of the Th1/Th2 response Th2 towards Th1 was observed at the high influenza antigen doses studied: such optimal doses would be expected to mask immunomodulation at the level of the Th1:Th2 response ratio.

### Example 4: Synthesis of 3,7-diepi-casuarine (10)

### General Experimental

All reactions were carried out under an atmosphere of argon at room temperature using anhydrous solvents unless otherwise stated. Anhydrous solvents were purchased from Fluka Chemicals and were used as supplied. Reagents were supplied from Aldrich, Fluka and Fisher and were used as supplied. Thin layer chromatography (TIc) was performed on aluminium sheets pre-coated with Merck 60 F₂₅₄ silica gel and where visualised under ultra-violet light and staining using 6% phosphomolybdic acid in ethanol. Silica gel chromatography was carried out using Sorbsil C60 40/60 silica gel under a positive atmosphere. Amberlite IR-120, strongly acidic ion-exchange resin was prepared by soaking the resin in 2M hydrochloric acid for at least two hours followed by elution with distilled water until the eluant reached pH 5. Dowex 50WX8-100 was prepared by soaking the resin with 2M hydrochloric acid for at least two hours followed by elution with distilled water until neutral. Infrared spectra were recorded on a Perkin-Elmer 1750 IR Fourier Transform spectrophotometer using thin films on sodium chloride plates. Only characteristic peaks are recorded. Optical rotations were measured on a Perkin-Eimer 241 polarimeter with a path length of 1dm. Concentrations are quoted in g/100mL. Nuclear magnetic resonance spectra were recorded on a Bruker DQX 400 spectrometer in the stated deuterated solvent. All spectra were recorded at ambient temperature. Chemical shifts (δ) are quoted in ppm and are relative to residual solvent as standard. Proton spectra (δ_{H}) were recorded at 400 MHz and carbon spectra (δ_{C}) at 100 MHz.

### 2,3:5,6:7,8-Tri-O-isopropylidene-D-erythro-L-talo-octono-1,4-lactone (Qc)

### 5,6:7,8-Di-O-isopropylidene-D-erythro-L-galacto-octono-1,4-lactone (Qb)

Sodium cyanide (7.02 g, 142 mmol) was added to a stirred solution of D-*glycero*-D-*gulo*-heptose (**Qa**, 21 g, 100 mmol) in water (300 ml). The reaction mixture was stirred at room temperature for 48 h, heated at reflux for 48 h and passed through a column containing Amberlite IR-120 (strongly acidic ion-exchange resin, 300 ml). The eluent was concentrated under reduced pressure and the residue dried *in vacuo* for 24 hours. The resulting foam was treated with acetone (500 ml) and sulphuric acid (5.4 ml) in the presence of anhydrous copper sulphate (10 g, 62 mmol) at room temperature for 48 h. T.I.c analysis Indicated the presence of two major products (ethyl acetate:cyclohexane, 1:1; R*_{f}* 0.72, 0.18). The reaction mixture was filtered and the filtrate was treated with sodium bicarbonate (50 g) for 24 h at room temperature. Solid residues were removed by filtration and the filtrate was concentrated under reduced pressure. The resulting crude yellow syrup was purified by silica gel chromatography providing 2,3:5,6:7,8-tri-*O*-isopropylidene-D-*erythro*-L-*talo*-actono-1,4-lactone **Qc** as a colourless syrup (R*_{f}* 0.72; 7.672 g; 21 %;) and 5,6:7,8,di-*O*-isopropylidene-D-*erythro*-L-*galacto*-octono-1,4-lactone **Qb** as a clear oil (R*_{f}* 0.18; 8.105 g; 25 %) 2,3:5,6:7,8-tri-*O*-isopropylidene-D-*erythro*-L-*talo*-octono-1,4-lactone **Qc** : δ_{H} (CDCl₃) 1.29, 1.33, 1.35, 1.38, 1.42, 1.48 (6 x s, 18H, 3 x C(C*H*₃)₂), 3.93-3.99 (m, 2H, *H*-8ₐ, *H-*7), 4.03-4.07 (m, 2H, *H*-5, *H*-6), 4.15 (dd, 1H, *J*_{8a,8b} 8.7 *J*_{8b,7} 6.1, *H*-8_{b}), 4.75-4.78 (m, 3H, H-2, H-3, H-4); δ_{c} (CDCl₃) 25.23, 25.51, 26.00, 26.71, 26.73, 27.16 (3 x C(*C*H₃)₂), 67.93, 74.93, 76.33, 76.69, 78.65, 79.40, 80.06, 109.95, 110.72, 113.19, 174.27; νₘₐₓ (film) 1793. 5,6:7,8-di-*O*-isopropylidene-D-*erythro*-L-*galacto*-octono-1,4-lactone **Qb** : δ_{H} (*d₆*-acetone) 1.28, 1.32, 1.34, 1.35 (4s, 12H, 2 x C(C*H*₃)₂), 3.92 (1 H, m, H-8ₐ), 3.98 (m, 1H, H-7), 4.14 (m, 2H, H-5, H-8_{b}), 4.23-4.25 (m, 2H, H-4, H-6), 4.35-4.40 (m, 2H, H-2, H-3); δ_{c} (*d₆*-acetone) 25.31, 25.87, 26.72, 27.31, 68.06, 75.15, 75.23, 77.51, 78.05, 78.41, 79.01, 110.06, 110.31, 174.25; νₘₐₓ (film) 1793, 3541.

### 2,3:5,6-Di-O-isopropylidene-D-erythro-L-talo-octono-1,4-lactone Qd

A solution of 2,3:5,6:7,8-tri-*O*-isopropylidene-D-*erythro*-L-*talo*-octono-1,4-lactone (Qc, 3.8 g, 10.6 mmol) was treated with acetic acid:water (2:3, 100 ml) at 50 °C for 2 h. T.I.c analysis (ethyl acetate:cyclohexane, 1:1) indicated the disappearance of the starting material (R*_{f}* 0.72) and the presence of a more polar compound (R*_{f}* 0.15). The solvent was removed under reduced pressure and the residue was purified by silica gel chromatography (ethyl acetae:cyclohexane, 1:1 to 3:1) yielding 2,3:5,6-di-*O*-isopropylidene-D-*erythro*-L-*talo-*octono-1,4-lactone **Qd** as a clear oil (3.23 g, 94 %): δ_{H} (CD₃OD) 1.28, 1.38, 1.43 (3 x s, 12H, 2 x C(C*H*₃)₂), 3.59 (dd, 1H, *J*_{8a,7} 5.40 *J*_{8a,8b} 11.41, *H*-8a), 3.66-3.69 (m, 1H, *H*-7), 3.74 (dd, 1H, *J*_{8b,7} 2.90 Hz, *H*-8_{b}), 4.01 (app t, 1H, *J*_{6,7} 7.62 Hz, H-6), 4.24 (dd, 1H, *J*_{5,6} 8.17 Hz *J*_{5,4} 0.89 Hz, H-5), 4.79-4.81 (m, 2H, *H*-3, *H-4*), 4.89-4.91 (m, 1 H, *H*-2); δ_{c} (CD₃OD) 24.62, 25.42, 26.05, 26.49, 63.86, 73.81, 75.40, 75.91, 79.18, 79.90, 80.78, 110.53, 113.09, 175.76; νₘₐₓ (film) 1791, 3478; [α]_{D} -35.7 (*c* 1, CHCl₃).

### 8-O-tert-Butyldimethylsilyl-2,3:5,6-di-O-isopropylidene-D-erythro-L-talo-octono-1,4-lactone Qe

To a solution of 2,3:5,6-di-*O*-isopropylidene-D-*erythro*-L-*talo*-octono-1,4-lactone (Qd, 3.18 g, 10 mmol) in *N*,*N-*dimethylformamide (40 ml) was added *tert*-butyldimethylsilyl chloride (1.808 g, 12 mmol) and imidazole (1.361 g, 20 mmol). The reaction mixture was stirred at room temperature for 16 h after which t.l.c. analysis (ethyl acetate:cyclohexane, 1:1) showed no starting material (R*_{f}* 0.15) and the formation of one major product (R*_{f}* 0.63). The solvent was removed under reduced pressure and the residue was partitioned between ethyl acetate and brine. The aqueous layer was extracted with ethyl acetate and the combined organic layers were dried (MgSO₄), filtered and the solvent removed. The resulting pale oil was purified by silica gel chromatography (ethyl acetate:cyclohexane, 0:1 to 1:2) to give 8-*O*-*tert*-butyldimethylsilyl-2,3:5,6-di-O-isopropylidene-D-*erythro-*L-*talo*-octono-1,4-lactone **Qe** as a clear oil (3.612 g, 85%): δ_{H} (CDCl₃) 0.04 (br s, 6H, 2 x C*H*₃), 0.86 (s, 9H, C(C*H*₃)₃), 1.23, 1.30, 1.32, 1.41 (4 x s, 12H, 2 x C(C*H*₃)₂), 3.63-3.67 (m, 2H, *H*-8ₐ, *H*-7), 3.76 (br d, 1H, *H*-8_{b}), 3.96 (app t, *J*_{6,7} 8.21 *J*_{6,5} 7.98, *H*-6), 4.08 (br d, 1H, *H*-5), 4.72 (br s, 2H, *H*-2, H-3), 4.78 (br s, 1H, H-4); δ_{c} (CDCl₃)-5.52, -5.45, 18.25, 25.51, 25.80, 25.93, 26.68, 27.18, 63.95, 72.97, 74.88, 74.93, 78.71, 79.63, 79.87, 110.34, 113.00, 174.42; νₘₐₓ (film) 1794, 3570; [α]_{D} -20.1 (c 1, CHCl₃).

### 7-Azido-8-O-tert-butyldimethylsilyl-7-deoxy-2,3:5,6-di-O-isopropylidene-L-threo-L-talo-octono-1,4-lactone Qf

A solution of 8-*O*-*tert*-butyldimethylsilyl-2,3:5,6-di-*O*-isopropylidene-D-*erythro*-L-*talo*-octono-1,4-lactone (**Qe**, 3.5 g, 8.2 mmol) in a pyrtdine:dichloromethane mixture (1:4, 25 ml) was cooled to -30 °C. Trifluoromethanesulfonic anhydride (3.5 g, 2.09 ml, 12.4 mmol) was added portion-wise and the mixture was stirred for 2 h. T.I.c analysis (ethyl acetate:cyclohexane, 1:3) indicated the disappearance of starting material (R*_{f}* 0.38) and the presence of a less polar product (R*_{f}* 0.48). The reaction mixture was concentrated under reduced pressure and the residue was partitioned between ethyl acetate and 0.5 M hydrochloric acid. The organic layer was washed with brine, dried (MgSO₄), filtered and concentrated under reduced pressure. The resulting crude pale orange residue was treated with sodium azide (807 mg, 12.4 mmol) in *N*,*N*-dimethylformamide (25 ml) for 16 h. T.I.c. analysis (ethyl acetate:cyclohexane, 1:4) indicated the disappearance of the intermediate triflate (R*_{f}* 0.42) and the presence of a more polar compound (R*_{f}* 0.40). The reaction solvent was removed *in vacuo* and the residue was partitioned between ethyl acetate and brine. The aqueous layer was extracted with ethyl acetate and the combined organic layers were dried (MgSO₄), filtered and concentrated *in vacuo.* The resulting crude residue was purified by silica gel chromatography (ethyl acetate:cyclohexane, 0:1 to 1:4) providing 7-azido-8-*O*-*tert*-butyldimethylsilyl-7-deoxy-2,3:5,6-di-*O*-isopropylidene-L-*threo*-L-*talo*-octono-1,4-lactone **Qf** as a colourless oil (3.026 g, 81%): δ_{H} (CDCl₃) 0.11 (2 x s, 6H, 2 x C*H*₃), 0.91 (s, 9H, C(C*H*₃)₃), 1.30, 1.38, 1.41, 1.47 (4 x s, 12H, 2 x C(C*H*₃)₂), 3.41-3.45 (m, 1H, H-7), 3.87 (dd, 1H, *J*_{8a,7} 5.37 Hz *J*_{8a,8b} 10.81 Hz, *H*-8ₐ), 3.92 (dd, 1H, *J*_{8b,7} 7.32 Hz, *H*-8_{b}), 4.19-4.24 (m, 2H, *H*-5, *H*-6), 4.61 (br s, 1H, *H*-4), 4.75-4.79 (m, 2H, H-2, H-3); δ_{c} (CDCl₃) -5.59, -5.56, 18.14, 25.54, 25.73, 26.09, 26.71, 26.98, 61.61, 63.19, 67.94, 74.84, 74.94, 75.47, 78.36, 78-66, 110.90, 113.37, 174.02; νₘₐₓ (film) 1796, 2111; [α]_{D} +36.7 (*c* 1, CHCl₃).

### 7-Azido-8-O-tert-butyldimethylsilyl-7-deoxy-2,3:5,6-di-O-isoprooylidene-L-threo-L-talo-octitol Qg

7-azido-8-*O*-*tert*-butyldimethylsilyl-7-deoxy-2,3:5,6-di-*O*-isopropylidene-L-*threo*-L-*talo*-octono-1,4-lactone (**Qf**, 3.00 g, 6.6 mmol) was dissolved in tetrahydrofuran (40 ml) and was cooled to 0 °C. Lithium borohydride (216 mg, 9.9 mmol) was added and the mixture was stirred at 0 °C to room temperature for 24 h. T.I.c. analysis (ethyl acetate:cyclohexane, 1:1) indicated the disappearance of the starting material (R*_{f}* 0.76) and the presence of a more polar compound (R*_{f}* 0.45). The reaction was quenched through the addition of ammonium chloride (sat. aq.) and the partitioned between ethyl acetate and brine. The aqueous layer was extracted with ethyl acetate (2 x) and the combined organic layers were dried (MgSO₄), filtered and the solvent removed. The resulting crude residue was purified by silica gel chromatography (ethyl acetate:cyclohexane, 1:3 to 1:1) affording 7-azido-8-*O*-*tert*-butyldimethylsilyl-7-deoxy-2,3:5,6-di-*O*-isopropylidene-L-*threo*-L-*talo*-octitol **Qg** as a colourless syrup (2.476 g, 82 %): δ_{H} (CDCl₃) 0.10 (s, 6H, 2 x C*H*₃), 0.91 (s, 9H, C(C*H*₃)₃), 1.36, 1.41, 1.42, 1.48 (4 x s, 12H, 2 x C(C*H*₃)₂), 3.43-3.47 (m, 1 H, H-7), 3.66 (br d, 1H, *H*-4), 3.79-3.92 (m, 4H, *H*-1, *H*-1ₐ, *H*-8, *H*-8ₐ), 4.10-4.14 (m, 2H, H-2, H-3), 4.30-4.38 (m, 2H, *H*-5, *H*-6); δ_{C} (CDCl₃) -5.61, -5.51, 18.14, 25.18, 25.71, 26.87, 27.07, 27.86, 60.65, 62.39, 63.66, 67.62, 75.90, 76.91, 77.18, 77.49, 108.63, 110.16; νₘₐₓ (film) 2109, 3536; [α]_{D} +46.6 (c 1, CHCl₃).

### 7-Azido-8-O-tert-butyldimethylsilyl-7-deoxy-2,3:5,6-di-O-isopropylidene-1,4-di-O-methanesulphonyl-L-threo-L-talo-octitol Qh

7-Azido-8-*O*-*tert*-butyldimethylsilyl-7-deoxy-2,3:5,6-di-*O*-isopropylidene-L-*threo*-L-*talo*-octitol (**Qg**, 2.4 g, 5.3 mmol) was dissolved in pyridine (20 ml) and was added to a solution of 4-dimethylamino pyridine (64 mg, 0.53 mmol) and methanesulfonyl chloride (4.814 g, 3.253 ml, 42 mmol) in pyridine (20 ml) and stirred for 2 h. T.I.c analysis (ethyl acetate:cyclohexane, 1:2, double elution) revealed the disappearance of starting material (R*_{f}* 0.33) and the presence of a more hydrophobic product (R*_{f}* 0.43). The solvent was removed under educed pressure and the residue was partitioned between ethyl acetate and brine. The aqueous layer was extracted with ethyl acetate and the combined organic layers were dried (MgSO₄), filtered and concentrated under reduced pressure. The resulting crude residue was purified by silica gel chromatography (ethyl acetate:cyclohexane, 1:2) giving 7-azido-8-*O*-*tert*-butyldimethylsilyl-7-deoxy-2,3:5,6-di-*O*-isopropylidene-1,4-di-*O*-methanesulfonyl-L-*threo*-L-*talo*-octitol **Qh** as a colourless oil (2.973 g, 92 %): δ_{H} (CDCl₃) 0.11, 0.12 (2 x s, 6H, 2 x C*H*₃), 0.91 (s, 9H, C(C*H*₃)₃), 1.41, 1.44, 1.46, 1.56 (4 x s, 12H, 2 x C(C*H*₃)₂), 3.08 (s, 3H, SO₂C*H*₃), 3.21 (s, 3H, SO₂C*H*₃), 3.49 (ddd, 1H, *J*_{7,6} 2.82 Hz, *J*_{7,8} 5.46 Hz, *J*_{7,8a} 7.94 Hz, *H*-7), 3.87-3.97 (m, 2H, H-8, *H*-8ₐ), 4.19 (dd, 1H, *J*_{6,5} 2.30 Hz, *H*-6), 4_{.}24-4.31 (m, 2H, H-1, *H*-5), 4.36 (dd, 1 H, *J*_{3,4} 2.96 Hz, *J*_{3,2} 6.62 Hz, *H*-3), 4.49-4.53 (m, 1H, *H*-2), 4.69 (dd, 1H, *J*_{1a,2} 2.39 Hz, *J*_{1a,1} 10.83 Hz, *H*-1a), 5.11 (app t, 1H, *H*-4); δ_{C} (CDCl₃) - 5.56, 18.18, 25.76, 26.24, 26.78, 26.89, 27.56, 37.75, 39.02, 60.90, 63.57, 70.44, 76.00, 76.07, 76.46, 77.18, 77.32, 109.01, 110.68; νₘₐₓ (film) 2113; [α]_{D} -16.2 (*c* 1, CHCl₃).

### 7-Azido-7-deoxy-1,4-di-O-methanesulphonyl-L-threo-L-talo-octitol Qi

7-Azido-8-*O*-*tert-*butyldimethylsilyl-7-deoxy-2,3:5,6-di-O-isopropylidene-1,4-di-O-methanesulfonyl-L-*threo*-L-talo-octitol (Qh, 2.90 g, 4.7 mmol) was treated with a trifluroacetic acid:water mixture (1:1, 40 ml) for 3 h. T.I.c. analysis (ethyl acetate) showed the disappearance of starting material (R*_{f}* 0.9) and the presence of a more polar product (R*_{f}* 0.12). The solvent was removed under reduced pressure and the residue was co-evaporated with toluene and dried under vacuum. Purification by silica gel chromatography (ethyl acetate:cylcohexane, 1:1 to 1:0) yielded 7-azido-7-deoxy-1,4-di-*O*-methanesulphonyl-L-*threo*-L-*talo*-octitol **Qi** as a colourless oil (1.677 g, 85 %): δ_{H} (CD₃OD) 3.12 (s, 3H, SO₂C*H*₃), 3.21 (s, 3H, SO₂C*H*₃), 3.61-3.71 (m, 2H, *H*-7, *H*-8), 3.78-3.82 (m, 2H, *H-*6, *H*-8ₐ), 3.98-4.05 (m, 2H, *H*-2, *H*-3), 4.11-4.13 (m, 1H, *H*-5), 4.34 (dd, 1H, *J*_{1,2} 4.87 Hz, *J*_{1,1a} 10.44 Hz, *H*-1), 4.45 (dd, 1H, *J*_{1a,2} 1.87 Hz, *H*-1ₐ), 5.00 (dd, 1H, *J*_{4.3} 1.91 Hz, *J*_{4,5} 6.15 Hz, *H*-4); δ_{C} (CD₃OD) 36.17, 38.11, 61.84, 66.62, 69.09, 70.33, 70.45, 71.08, 72.55, 86.41; νₘₐₓ (film) 2113; [α]_{D}-9.1 (*c* 1, H₂O).

### (1R,2R,3S,6S,7R,7aR)-3-(Hydroxymethyl)-1,2,6,7-tetrahydroxypyrrolizidine Qj [3,7-diepi-Casuarine]

7-Azido-7-deoxy-1,4-di-*O*-methanesulphonyl-L-*threo*-L-*talo*-octitol (**Qi**, 1.6 g, 3.78 mmol) was dissolved in water (30 ml) and was treated with 10 % palladium on carbon (400 mg) under an atmosphere of hydrogen for 16 h. T.I.c analysis (ethyl acetate:methanol, 9:1) indicated the disappearance of starting material (R*_{f}* 0.75) and the presence of a more polar product (R*_{f}* 0.05). Palladium was removed by filtration and the filtrate was treated with sodium acetate (930 mg, 11.34 mmol) at 60 °C for 16 h. The reaction mixture was cooled and the solvent removed in *vacuo.* The crude brown oil was purified by ion-exchange chromatography (Dowex 50WX8-100, eluting with 2M ammonium hydroxide) to afford (1R,2R,3S,6S,7R,7aR)-3-(hydroxymethyl)-1,2,6,7-tetrahydroxypyrrolizidine [3,7-*diepi*-Casuarine] **Qj** as a brown glass (671 mg, 87%): δ_{H} (D₂O) 2.81-2.92 (m, 2H, H-5, *H*-5ₐ), 3.16 (dd, 1H, *J*_{3,2} 5.91 Hz, *J*_{3,8} 10.74 Hz, *H*-3), 3.30 (app t, 1 H, J 3.78 Hz, *H*-7ₐ), 3.76 (dd, 1H, *J*_{8,8a} 6.35 Hz, *H*-8), 3.87 (dd, 1H, *H*-8ₐ), 4.01 (d, 1H, *J*_{2,1} 3.55 Hz, *H*-2), 4.04-4.12 (m, 2H, *H*-6, *H*-7), 4.29 (app t, 1H, *H*-1); δ_{c} (D₂O) 49.32, 57.29, 63.78, 70.41, 72.59, 72.65, 74.47, 78.25; [α]_{D} -21.1 (c 0.5, H₂O).

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. Use of one or more antigen(s) and an adjuvant composition comprising a Th1-activating alkaloid for the manufacture of a vaccine for use in vaccination for polarizing an immune response to the antigen(s) from type 2 towards type 1, wherein the alkaloid has the formula: 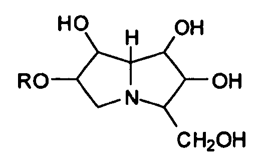 wherein R is selected from the group comprising hydrogen, straight or branched, unsubstituted or substituted, saturated or unsaturated acyl, alkyl for example cycloalkyl, alkenyl, alkynyl and aryl groups, or a pharmaceutically acceptable salt or acyl derivative thereof.

2. Use of claim 1 wherein the Th1-activating alkaloid stimulates the expression of IL-12 *in vitro* in lymphocytes and/or dendritic cells.

3. Use of claim 1 or claim 2 wherein the adjuvant composition further comprises an auxiliary adjuvant, for example an auxiliary adjuvant selected from:
(a) a type 2 adjuvant for example alum and/or MF59; and/or
(b) a cytokine;
(c) a depot-forming agent;
(d) a saponin;
(e) a submicron oil-in-water emulsion;
(f) a CpG;
(g) a lipid A derivative;
(h) an MDP;
(i) an ISCOM®;
(j) an antigen-presenting cell (APC) for example a dendritic cell;
(k) a cytotoxic T lymphocyte (CTL); and
(l) a synergistic combination of any of the above.

4. Use of any one of the preceding claims wherein the vaccination comprises the administration of a vaccine selected from: (a) a subunit vaccine; (b) a conjugate vaccine; (c) a DNA vaccine; (d) a recombinant vaccine; (e) a mucosal vaccine; (f) a therapeutic vaccine; (g) a prophylactic vaccine.

5. Use of any one of the preceding claims wherein the one or more antigen(s) are selected from:
(a) nucleic acid(s) which encode one or more antigenic protein(s);
(b) protein(s) or peptide(s);
(c) glycoprotein(s);
(d) polysaccharide(s) for example carbohydrate(s);
(e) fusion protein(s);
(f) lipid(s);
(g) glycolipid(s);
(h) peptide mimic(s) of polysaccharides;
(i) carbohydrate(s) and a protein(s) in admixture;
(j) carbohydrate-protein conjugate(s);
(k) cells or extracts thereof;
(l) dead or attenuated cells, or extracts thereof;
(m) tumour cells or extracts thereof;
(n) viral particles for example attenuated viral particles or viral components;
(o) allergen(s);
(p) mixtures of any of (a) to (o).

6. Use of claim 5 wherein the one or more antigen(s) comprise a bacterial antigen, a viral antigen, a fungal antigen, a protozoal antigen, a prion antigen, a neoantigen, a tumour-associated antigen or a self-antigen.

7. Use of claim 5 or claim 6 wherein the one or more antigen(s) are dose-spared.

8. Use of any one of the preceding claims wherein the vaccination is conducted orally, mucosally, topically, epicutaneously, intramuscularly, intradermally, subcutaneously, intranasally, intravaginally, sublingually or via inhalation.

9. Use of any one of the preceding claims wherein the Th-1 activating alkaloid is selected from:
(a) 3,7-*diepi*-casuarine;
(b) 7-*epi*-casuarine;
(c) 3,6,7-*triepi*-casuarine;
(d) 6,7-*diepi*-casuarine;
(e) 3-*epi*-casuarine;
(f) 3,7-*diepi*-casuarine-6-α-D-glucoside;
(g) 7-*epi*-casuarine-6-α-D-glucoside;
(h) 3,6,7-*triepi*-casuarine-6-α-D-glucoside;
(i) 6,7-*diepi*-casuarine-6-α-D-glucoside; and
(j) 3-*epi*-casuarine-6-α-D-glucoside,
or a pharmaceutically acceptable salt or acyl derivative thereof.

10. Use of any one of the preceding claims wherein the Th-1 activating alkaloid is 3,7-diepicasuarine having the formula: 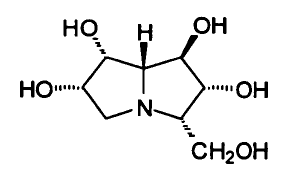 or a pharmaceutically acceptable salt or acyl derivative thereof.

## Patentansprüche

1. Verwendung eines Antigens oder mehrerer Antigene und einer Adjuvanszusammensetzung, umfassend ein Th1-aktivierendes Alkaloid für die Herstellung eines Impfstoffs für den Einsatz zur Immunisierung, um eine Immunantwort vom Typ 2 in Richtung Typ 1 gegen das Antigen/die Antigene zu polarisieren, wobei das Alkaloid die Formel 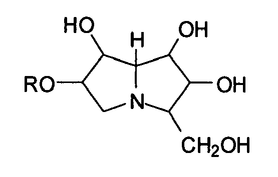 aufweist, wobei R ausgewählt ist aus der Gruppe, welche Wasserstoff, geradkettiges oder verzweigtes, unsubstituiertes oder substituiertes, gesättigtes oder ungesättigtes Acyl, Alkyl, zum Beispiel Cycloalkyl-, Alkenyl-, Alkynyl- und Arylgruppen, oder ein pharmazeutisch akzeptables Salz oder Acylderivat davon umfasst.

2. Verwendung nach Anspruch 1, wobei das Th1-aktivierende Alkaloid die Expression von IL-12 *in vitro* in Lymphozyten und/oder dendritischen Zellen stimuliert.

3. Verwendung nach Anspruch 1 oder Anspruch 2, wobei die Adjuvanszusammensetzung des Weiteren ein zusätzliches Adjuvans umfasst, zum Beispiel ein zusätzliches Adjuvans, ausgewählt aus:
(a) einem Typ-2-Adjuvans, zum Beispiel Alum und/oder MF59, und/oder
(b) einem Cytokin,
(c) einem Depot-bildenden Agens,
(d) einem Saponin,
(e) einer submikronen Öl-in-Wasser-Emulsion,
(f) einem CpG,
(g) einem Lipid-A-Derivat,
(h) einem MDP,
(i) einem ISCOM®;
(j) einer Antigen-präsentierenden Zelle (APC), zum Beispiel einer dendritischen Zelle,
(k) einem zytotoxischen T-Lymphozyten (CTL) und
(l) einer synergistischen Kombination der oben Erwähnten.

4. Verwendung nach einem der vorhergenden Ansprüche, wobei die Immunisierung die Verabreichung eines Impfstoffs umfasst, ausgewählt aus: (a) einem Subunit-Impfstoff, (b) einem Konjugat-Impfstoff, (c) einem DNA-Impfstoff, (d) einem rekombinanten Impfstoff, (e) einem mukosalen Impfstoff; (f) einem therapeutischen Impfstoff, (g) einem prophylaktischen Impfstoff.

5. Verwendung nach einem der vorhergehenden Ansprüche, wobei das eine oder die mehreren Antigen(e) ausgewählt ist/sind aus :
(a) Nukleinsäure(n), welche für ein oder mehrere antigene(s) Protein(e) kodieren,
(b) Protein(e) oder Peptide(e),
(c) Glykoprotein(e),
(d) Polysaccharid(e) zum Beispiel Kohlenhydrat(e),
(e) Fusionsprotein(e),
(f) Lipid(e),
(g) Glykolipid(e),
(h) Peptidmimetikum(-mimetika) von Polysacchariden,
(i) Kohlenhydrat(e) und ein Protein(e) als Zusatz,
(j) Kohlenhydrat-Protein-Konjugat(e),
(k) Zellen oder Extrakte davon,
(l) tote oder abgeschwächte Zellen oder Extrakte davon,
(m) Tumorzellen oder Extrakte davon,
(n) virale Partikel, zum Beispiel abgeschwächte virale Partikel oder virale Bestandteile,
(o) Allergen(e),
(p) Mischungen aus (a) bis (o).

6. Verwendung nach Anspruch 5, wobei das eine oder die mehreren Antigen(e) ein bakterielles Antigen, ein virales Antigen, ein Pilzantigen, ein Protozoenantigen, ein Prionantigen, ein Neoantigen, ein Tumor-assoziiertes Antigen oder ein Selbst-Antigen umfasst/umfassen.

7. Verwendung nach Anspruch 5 oder Anspruch 6, wobei das eine oder die mehreren Antigen(e) dosissparend ist/sind.

8. Verwendung nach einem der vorhergehenden Ansprüche, wobei die Impfung oral, mukosal, topisch, epikutan, intramuskulär, intradermal, subkutan, intranasal, intravaginal, sublingual oder mittels Inhalation durchgeführt wird.

9. Verwendung nach einem der vorhergehenden Ansprüche, wobei das Th-1 aktivierende Alkaloid ausgewählt ist aus:
(a) 3,7-*diepi*-Casuarin,
(b) 7-*epi*-Casuarin,
(c) 3,6,7-*triepi*-Casuarin,
(d) 6,7-*diepi*-Casuarin,
(e) 3-*epi*-Casuarin,
(f) 3,7-*diepi*-Casuarin-6-α-D-Glucosid,
(g) 7-*epi*-Casuarin-6-α-D-Glucosid,
(h) 3,6,7-*triepi*-Casuarin-6-α-D-Glucosid,
(i) 6,7-*diepi*-Casuarin-6-α-D-Glucosid und
(j) 3-*epi*-Casuarin-6-α-D-Glucosid
oder einem pharmazeutisch akzeptablen Salz oder Acylderivat davon.

10. Verwendung nach einem der vorhergehenden Ansprüche, wobei es sich bei dem Th-1 aktivierenden Alkaloid um 3,7-*diepi*-casuarin mit der Formel 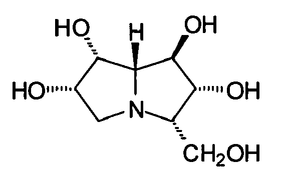 oder ein pharmazeutisch akzeptables Salz oder Acylderivat davon handelt.

## Revendications

1. Utilisation d'un ou plusieurs antigènes et d'une composition adjuvante comprenant un alcaloïde activateur des Th1 dans la fabrication d'un vaccin conçu pour être utilisé dans un processus de vaccination permettant de polariser une réponse immunitaire à l'antigène ou aux antigènes de type 2 au profit d'une réponse immunitaire de type 1, ledit alcaloïde répondant à la formule : 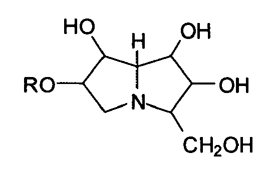 où R est sélectionné dans le groupe comprenant hydrogène, un groupement acyle saturé ou insaturé et linéaire ou ramifié qui est substitué ou non, un groupement alkyle tel qu'un groupement cycloalkyle ou des groupements alcényles, alcynyles et aryles, ou un sel ou un dérivé à radical acyle pharmaceutiquement acceptable de celui-ci.

2. Utilisation selon la revendication 1, où l'alcaloïde activateur des Th1 stimule l'expression d'IL-12 dans des lymphocytes et/ou des cellules dendritiques *in vitro.*

3. Utilisation selon la revendication 1 ou de la revendication 2, où la composition adjuvante comprend également un adjuvant auxiliaire, par exemple un adjuvant auxiliaire sélectionné parmi les suivants :
(a) un adjuvant favorisant une réponse de type 2, par exemple l'alum et/ou MF59 ; et/ou
(b) une cytokine ;
(c) un agent formant un dépôt ;
(d) une saponine ;
(e) une émulsion d'huile dans l'eau submicronique ;
(f) un oligonucléotide à motif CpG ;
(g) un dérivé du lipide A
(h) un MDP ;
(i) un complexe ISCOM^{®} ;
(j) une cellule présentatrice d'antigènes (APC), par exemple une cellule dendritique ;
(k) un lymphocyte T cytotoxique (CTL) ; et
(l) une combinaison synergique de certains ou de tous des composants répertoriés ci-dessus.

4. Utilisation selon l'une quelconque des revendications précédentes, où la procédure de vaccination comprend l'administration d'un vaccin sélectionné parmi : (a) un vaccin sous-unitaire ; (b) un vaccin conjugué ; (c) un vaccin à ADN ; (d) un vaccin recombinant ; (e) un vaccin muqueux ; (f) un vaccin thérapeutique ; (g) un vaccin prophylactique.

5. Utilisation selon l'une quelconque des revendications précédentes, où lesdits un ou plusieurs antigènes sont sélectionnés parmi les suivants :
(a) acide(s) nucléique(s) codant pour une ou plusieurs protéine(s) antigénique(s) ;
(b) protéine(s) ou peptide(s) ;
(c) glycoprotéine(s) ;
(d) polysaccharide(s), par exemple un ou des hydrate(s) de carbone ;
(e) protéine(s) de fusion ;
(f) lipide(s) ;
(g) glycolipide(s)
(h) mimétique(s) peptidique(s) de polysaccharides
(i) hydrate(s) de carbone et protéine(s) en mélange ;
(j) conjugué(s) hydrate de carbone-protéine ;
(k) cellules ou extraits cellulaires ;
(l) cellules tuées ou atténuées, ou extraits de telles cellules ;
(m) cellules tumorales ou extraits de telles cellules ;
(n) particules virales, par exemple des particules virales ou des composants viraux atténués ;
(o) allergène(s) ;
(p) mélanges de certains ou plusieurs des composants (a) à (o).

6. Utilisation selon la revendication 5, où lesdits un ou plusieurs antigènes comprennent un antigène bactérien, un antigène viral, un antigène fongique, un antigène protozoaire, une protéine prion immunogène, un néoantigène, un antigène associé à une tumeur et un autoantigène.

7. Utilisation selon la revendication 5 ou la revendication 6, où la quantité desdits un ou plusieurs antigènes utilisée dans la dose est réduite.

8. Utilisation selon l'une quelconque des revendications précédentes, où la procédure de vaccination est effectuée par voie orale, muqueuse, locale, épicutanée, intramusculaire, intradermique, sous-cutanée, intranasale, intravaginale, sublinguale ou inhalée.

9. Utilisation selon l'une quelconque des revendications précédentes, où l'alcaloïde activateur des Th1 est sélectionné parmi les suivants :
(a) 3,7-di-*épi*-casuarine ;
(b) 7-*épi*-casuarine ;
(c) 3,6,7-tri-*épi*-casuarine ;
(d) 6,7-di-*épi*-casuarine ;
(e) 3-*épi*-casuarine ;
(f) 3,7-di-*épi*-casuarine-6-α-D-glucoside ;
(g) 7-*épi*-casuarine-6-α-D-glucoside ;
(h) 3,6,7-tri-*épi*-casuarine-6-α-D-glucoside ;
(i) 6,7-di-*épi*-casuarine-6-α-D-glucoside ; et
(j) 3-*épi*-casuarine-6-α-D-glucoside,
ou un sel ou un dérivé à radical acyle pharmaceutiquement acceptable de celui-ci ;

10. Utilisation selon l'une quelconque des revendications précédentes, où l'alcaloïde activateur des Th1 est la 3,7-di-*épi*-casuarine de formule : 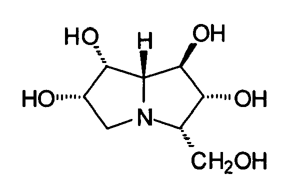 ou un sel ou un dérivé à radical acyle pharmaceutiquement acceptable de celui-ci.
